# EUROPEAN PATENT SPECIFICATION

(11) **EP 4 568 725 B1**
(45) Date of publication and mention of the grant of the patent: **17.09.2025**
(21) Application number: 23822007.3
(22) Date of filing: 12.12.2023
(51) Int. Cl.: A61M 16/00

(54) **CPAP THERAPY ADAPTATION SYSTEM TO MITIGATE PULMONARY HYPERINFLATION**
ANPASSUNGSYSTEM FÜR CPAP THERAPIE ZUM MILDERN PULMONALER HYPERINFLATION
SYSTÈME D'ADAPTATION DE THÉRAPIE CPAP POUR ATTÉNUER L'HYPERINFLATION PULMONAIRE

(30) Priority: 23.12.2022 US 202263434964 P
(43) Date of publication of application: 18.06.2025
(73) Proprietor: Koninklijke Philips N.V., 5656 AG Eindhoven (NL)
(72) Inventor: HENDRIKS, Cornelis Petrus, 5656 AG Eindhoven (NL); KAHLERT, Joachim Johannes, 5656 AG Eindhoven (NL); VAN ZANTEN, Joyce, 5656 AG Eindhoven (NL); DOS SANTOS DA FONSECA, Pedro Miguel Ferreira, 5656 AG Eindhoven (NL)
(74) Representative: Philips Intellectual Property & Standards
(86) International application number: PCT/EP2023/085250
(87) International publication number: WO 2024/132661

(56) References cited:
- EP-B1- 2 943 121
- WO-A1-2017/029629
- US-A1- 2008 302 364
- US-A1- 2016 361 041
- US-A1- 2020 100 728
- US-A1- 2021 393 902
- US-A1- 2022 395 653
- US-B1- 11 285 286
- US-B1- 6 588 422

## Description

### CROSS REFERENCE TO RELATED APPLICATIONS

This application claims the benefit to European Application Ser. No. 22214321.6, filed on December 16, 2022, entitled DETECTING, IDENTIFYING AND ALLEVIATING CAUSES FOR PRESSURE BURDEN USING POSITIVE AIRWAY PRESSURE DEVICES, (Docket No. 2022P00891EP).

### FIELD OF THE PRESENT SYSTEM

The present system relates to a continuous positive airway pressure (CPAP) device with hyperinflation, arousal, and airway pressure/resistance sensing (i.e., flow) and, more particularly, to a CPAP system with hyperinflation, arousal, and airway pressure/resistance sensing, and a controller with learning and a feedback loop for optimal and desirable personalized pressure adaptation to improve both apnea hypopnea index (AHI) and sleep quality of individuals receiving therapy from the CPAP system, and a method of operation thereof.

### BACKGROUND OF THE PRESENT SYSTEM

Hyperinflation occurs in patients under positive airway pressure (PAP) therapy for many reasons. Due to the positive airway pressure, an extrinsic positive expiratory pressure is generated by the PAP device, which leads to an inflation of a chest of a person or subject (e.g., a patient) receiving the PAP therapy. When the PAP pressure settings are increased from about 4 through 8 cmH₂O (e.g., 6mbar), breathing effort typically switches from passive expiration to active expiration. However, this range may vary and may be lower in some comorbidities and seasonal infections. Active expiration requires muscular breathing effort which effort increases the outflow in expiration and counteracts inflation caused by the pressure from the PAP device. However, in deep-sleep, the ability (e.g., the muscular effort and/or work of breathing declines) to actively expire declines, which can cause of hyperinflation during sleep. Another cause of hyperinflation is chronic obstructive pulmonary disease (COPD) comorbidity which induces an intrinsic positive end expiratory pressure (PEEP) due to stiff and narrow central airways and/or lung tissue and gas trapping.

Hyperinflation can have many adverse consequences. For example, these consequences of hyperinflation can cause increased thoracic pressure and an inflated chest can activate baroreceptors and stretch receptors, which lead to an elevated sympathetic outflow. Other consequences of hyperinflation can cause increased intrathoracic pressure and an increases positive end expiratory pressure (PEEP). The intrathoracic pressure impedes the cardiac flow parameter and can impede the venous return flow (VRF) from the peripheral veins into the right atrium. The resultant reduced filling of the right atrium (RA) reduces the pulmonary circulation and consequently the stroke volume (SV) of the left ventricle (LV). The change in the atrial filling is sensed by baroreceptors/stretch receptor in the atrium. The activation of the baroreceptors can initiate a sympathetic outflow. The response to the changed sympathetic activity is an electrophysical alteration, a change of heart rate (HR), a change of respiration and heart rate variability (HRV). With regard to the stroke volume (SV), a change of the SV can be monitored in the systemic arteries, for instance by photoplethysmogram (PPG) sensor attached to or located at a finger of a patient. The SV change impedes the amplitude of the PPG signal, which is specific for an SV change. The increased sympathetic activity is known to cause arousals, increased heart rate, increased blood pressure, perceived discomfort, impression of therapy non-efficacy, and/or non-adherence to CPAP therapy. Uncontrolled hyperinflation can also affect heart function and can contribute to, or cause, heart failure in the long term. Further, the induced higher intrathoracic pressure of hyperinflation can modulate cardiac preload/afterload and can impede venous return flow and stroke volume. This volume shift within the atria can cause undue stretching of the musculature within. This undue stretching can lead to conduction anomalies seen in the setting of cardiac arrythmias (e.g., atrial fibrillation and/or premature contractions).

PCT application WO 2017/029629A1 discloses a resuscitator apparatus that receives patient details, and controls a motor to move a piston to deliver an initial tidal volume during an initial respiratory cycle that is smaller than a best practice tidal volume associated with said patient details. US patent application US 2021/393902 discloses a system for one-touch ventilation mode. Settings for a medical ventilator are determined and delivered to a patient with a minimum of one input parameter. Based on the ventilation data, one or more ventilation strategies may be implemented, including breath type strategy, alarming strategy, triggering/cycling strategy, and PEEP strategy. US patent application US 2022/395653 A1 discloses a monitor for evaluating airway procedures, particularly in a pre-hospital environment. An airway parameter of an individual receiving assisted ventilation is detected by an airway sensor. A monitor determines a metric based on the airway sensor. Further, the monitor performs an action based on the metric.

### SUMMARY OF THE PRESENT SYSTEM

While some chest inflation under CPAP therapy may be unavoidable, it is important to detect an increasing level of hyperinflation and more importantly, a maximal tolerable or upper boundary for chest inflation for each patient receiving CPAP therapy. However, as this depends on the physiological implications and comorbidities of each patient, the maximal tolerable or upper boundary for chest inflation for each patient can change. For example, embodiments of the present system may determine when is the patient not able to increase breathing effort during expiration to expire actively, when is the patient aroused, and/or when does the heart function deteriorate too much. Additionally, there is a need to provide decision support to either mitigate or reverse the chest inflation by compensating for the inability to expire by changing PAP device therapy settings. This can be of great importance particularly in the context of OSA-COPD overlap syndrome patients, where many factors interact in a complex and sometimes opposing manner in the relations between hyperinflation, increased sympathetic activity, airway resistance/patency and pressure therapy. For example, while (a moderate) hyperinflation can have a positive effect on the severity and implications of obstructive sleep apnea (OSA) due to airway stiffening and/or narrowing which can reduce AHI, hyperinflation and the changed sympathetic activity can have a negative effect due to lowering the arousal threshold and decreasing sleep quality. Similarly, positive airway pressure (PAP) can have a positive effect on the severity and implications of OSA and COPD by keeping the upper and central airways open and promoting lung deflation (e.g., by lowering airway resistance), PAP can also have a negative effect by promoting hyperinflation.

Thus, there is a need for a ventilation support therapy (VST) adaptation, such as a CPAP therapy adaptation, to mitigate or reverse pulmonary hyperinflation for patients using ventilation support to control risk factors which may lead to poor clinical outcomes when using ventilation support therapy (VST), such as PAP therapy, CPAP therapy and/or the like. These patients may or may not have comorbidities such as one or more of COPD, and/or congestive heart failure (CHF). There is also a need to monitor hyperinflation and to offer a personalized pressure adaptation for each patient.

Accordingly, embodiments of the present system provide features and advantages which obviate conventional PAP systems and methods. To overcome the aforementioned barriers and detriments as well as others, there is a need for a system which can provide patients who may have complicated comorbidities to use sensor-mediated control to control hyperinflation caused by PAP therapy in an automated fashion and to mitigate cardiac risk factors (e.g., for patients with comorbidities) as well as increase sleep quality, and therapy compliance (e.g., for all patient). This can also free physicians from having to monitor at risk patients when using CPAP therapy operating in accordance with embodiments of the present system thus overcoming the aforementioned barriers and detriments as well as others of conventional systems.

The present system(s), device(s), method(s), arrangements(s), interface(s), computer program(s), processes, etc., (hereinafter each of which will be referred to as system, unless the context indicates otherwise), described herein address problems in prior art systems.

In accordance with embodiments of the present system, there is disclosed a positive airway pressure (PAP) therapy device, including one or more of: at least one controller; a flow generator configured to provide an airflow to a user under control of the at least one controller; a plurality of sensors including at least one electrocardiogram (ECG) sensor, at least one photoplethysmogram (PPG) sensor, and/or at least one flow sensor, wherein the at least one flow sensor is configured to determine inspiratory and expiratory flow patterns and form corresponding inspiratory and expiratory flow information, and wherein the at least one ECG sensor is configured to sense physiological parameters of a user and form corresponding ECG sensor information; wherein the at least one PPG sensor is configured to measure variability in stroke volume (SV), ejection fraction (EF), and/or cardiac output (CO); the at least one controller configured to: control the flow generator to provide therapeutic air assist to the user in accordance with an initial setting mode; distinguish active and passive expiration based on the inspiratory and expiratory flow information; determine a rise time of expiration (RTE) based upon the expiratory flow information, wherein the rise time of expiration (RTE) is indicative of how fast the user exhales, wherein the shorter the expiration rise time (RTE) the stronger the hyperinflation; compare the rise time of expiration (RTE) to a threshold rise time of expiration value (Trte) to determine whether the rise time of expiration (RTE) is less than the threshold rise time of expiration value (Trte); determine hyperinflation when the at least one controller determines that the rise time of expiration (RTE) is less than the threshold rise time of expiration value (Trte); following determining the hyperinflation, determine arousals based on the ECG sensor information; determine a decreased cardiac output or stroke volume and/or ejection fraction following determining the hyperinflation; determine the hyperinflation is critical based on the rise time of expiration (RTE) being less than the threshold rise time of expiration value (Trte), and the arousals, and the decreased cardiac output or stroke volume and/or ejection fraction; control the flow generator to operate in accordance with a hyperinflation mode to reduce therapeutic air assist to the user when the at least one controller determines that the hyperinflation is critical; and control the flow generator (144) to decrease expiration pressure by a threshold pressure value when the PAP therapy device is in the hyperinflation

The aforementioned positive airway pressure (PAP) therapy device forms the subject-matter of the appended independent claim 1. Further embodiments of said (PAP) therapy device are defined in the appended dependent claims.

In accordance with embodiments of the present system, the at least one controller may be further configured to control the flow generator to reduce at least one of Bi-flex, C-flex, and A-flex settings of the PAP therapy device by a threshold pressure value when in the hyperinflation mode. It is also envisioned the at least one controller may be configured to control the flow generator to decrease duration of pressure support during inspiration cycle for N breath cycles by a threshold time value when in the hyperinflation mode, where N is an integer. In some embodiments of the present system, the at least one controller may be further configured to control the flow generator to decrease expiration pressure by a threshold pressure value when the PAP therapy device is in one of the hyperinflation modes and in a BiPAP therapy mode. The at least one controller may be further configured to control the flow generator to reduce at least one of duration of pressure support during inspiration cycle and inspiration pressure by corresponding threshold amounts when in the hyperinflation mode. The plurality of sensors may include one or more of a chest sensors and an abdominal area sensor configured to measure a breathing pattern and extension of a chest of the user and form corresponding chest inflation sensor information (CISI). It is envisioned that the at least one controller may be further configured to compare the chest inflation sensor information (CISI) to a threshold chest inflation value (Tci). When in the hyperinflation mode, the at least one controller may be configured to determine the hyperinflation is not critical when the CISI is determined to be less than the Tci.

It is envisioned that the at least one controller may be further configured to control the flow generator to return to operation in accordance with the initial setting mode to restore therapeutic air assist to the user in accordance with the initial setting mode, when the at least one controller determines that the hyperinflation is not critical. The at least one controller may be further configured to save in a memory current hyperinflation mode operating parameters of the flow generator prior to returning to operation in accordance with the initial setting mode from the hyperinflation mode. When the at least one controller determines that the hyperinflation is critical, the at least one controller is further configured to change operating parameters of the flow generator to the saved current hyperinflation mode operating parameters. The at least one controller may be further configured to determine the hyperinflation is not critical when determining that the rise time of expiration (RTE) is not less than the threshold rise time of expiration (Trte). It is also envisioned that the at least one controller may be further configured to control the flow generator to return to operation in accordance with the initial setting mode to restore therapeutic air assist to the user in accordance with the initial setting mode, when the at least one controller determines that hyperinflation is not critical. The at least one controller may be further configured to obtain PPG sensor information from the at least one PPG sensor and determine variability in stroke volume and ejection fraction and form corresponding stroke volume (SV) and ejection fraction (EF) information.

It is also envisioned that the at least one controller may be further configured to restore current pressure values to the initial pressure values when the at least one controller determines that the venous return flow (VRF) is not less than the threshold for venous return flow (Tvrf) or the stroke volume (SV) is not less than the threshold for stroke volume (Tsv).

In accordance with embodiments of the present system, there is disclosed a positive airway pressure (PAP) therapy device, which may include one or more of: at least one controller; a plurality of sensors including at least one electrocardiogram (ECG) sensor, at least one photoplethysmogram (PPG), and at least one flow sensor, the at least one flow sensor configured to determine inspiratory and expiratory flow and form corresponding inspiratory and expiratory flow information, and the at least one ECG sensor configured to sense physiological parameters of a user and form corresponding ECG sensor information; the at least one controller may be configured to: control a flow generator to provide therapeutic air assist to the user in accordance with an initial setting mode; determine a rise time of expiration (RTE) based upon the expiratory flow information; compare the rise time of expiration (RTE) to a threshold rise time of expiration value (Trte) to determine whether the rise time of expiration (RTE) is less than the threshold rise time of expiration value (Trte);determine hyperinflation is critical when the at least one controller determines that the rise time of expiration (RTE) is less than the threshold rise time of expiration value (Trte); and may control the flow generator to operate in accordance with a hyperinflation mode to reduce therapeutic air assist to the user when the at least one controller determines that the hyperinflation is critical.

It is also envisioned that the at least one controller may be further configured to obtain PPG sensor information from the at least one PPG sensor and determine variability in stroke volume and ejection fraction and form corresponding stroke volume (SV) and ejection fraction (EF) information. The at least one controller may be further configured to control the flow generator to reduce at least one of Bi-flex, C-flex, and A-flex settings of the PAP therapy device by a threshold pressure value when in the hyperinflation mode.

It is further envisioned that the least one controller may be further configured to control the flow generator to decrease duration of pressure support during inspiration cycle for N breath cycles by a threshold time value when in the hyperinflation mode, where N is an integer.

### BRIEF DESCRIPTION OF THE DRAWINGS

It should be expressly understood that the drawings are included for illustrative purposes and do not represent the scope of the present system. It is to be understood that the figures may not be drawn to scale. Further, the relation between objects in a figure may not be to scale and may in fact have a reverse relationship as to size. The figures are intended to bring understanding and clarity to the structure of each object shown, and thus, some features may be exaggerated in order to illustrate a specific feature of a structure. In the accompanying drawings, like reference numbers in different drawings may designate identical or similar elements, portions of similar elements and/or elements with similar functionality. The present system is explained in further detail, and by way of example, with reference to the accompanying drawings which show features of various exemplary embodiments that may be combinable and/or severable wherein:
FIG. 1 illustratively shows a schematic block diagram of a portion of a system operating in accordance with embodiments of the present system;
FIG. 2 illustratively shows a functional flow diagram performed by a process performed for a patient with Sleep disordered breathing (SDB) patient in accordance with embodiments of the present system;
FIG. 3 illustratively shows a functional flow diagram performed by a process for COPD and OSA comorbid patients in accordance with embodiments of the present system;
FIG. 4 illustratively shows a functional flow diagram performed by a process for resolving diminished cardiac output in response to ASV therapy with SDB patients comorbid with CHF in accordance with embodiments of the present system; and
FIG. 5 illustratively shows a functional flow diagram performed by a process for assisting decision support to adapt PAP therapy accordance with embodiments of the present system.

### DETAILED DESCRIPTION OF THE PRESENT SYSTEM

The following are descriptions of illustrative embodiments that when taken in conjunction with the following drawings will demonstrate the above noted features and advantages, as well as further ones. In the following description, for purposes of explanation rather than limitation, illustrative details are set forth such as architecture, interfaces, techniques, element attributes, etc. However, it will be apparent to those of ordinary skill in the art that other embodiments that depart from these details would still be understood to be within the scope of the appended claims. Moreover, for the purpose of clarity, detailed descriptions of well-known devices, circuits, tools, techniques, and methods are omitted so as not to obscure the description of the present system.

The term "and/or," and formatives thereof, should be understood to mean that only one or more of: the recited elements may need to be suitably present (e.g., only one recited element is present, two of the recited elements may be present, etc., up to all of the recited elements may be present) in a system in accordance with the claims recitation and in accordance with one or more embodiments of the present system. In the context of the present embodiments, the terms "about", substantially and "approximately" denote an interval of accuracy that a person skilled in the art will understand to still ensure the technical effect of the feature in question which in some cases may also denote "within engineering tolerances." For example, the terms may indicate a deviation from the indicated numerical value(s) of ±20 %, ±15 %, ±10 %, ±5 %, ±1 % ±0.5 % or ±0.1 %.

The terms clinician, clinicians, and/or formatives thereof, may include a professional such as a doctor, a clinician, a technologist, an operator, an expert, medical specialist, a technician, and/or the like, who may operate and/or review information related to the patient such as system setting information (SSI), system information, and/or data generated by embodiments of the present system and associated data for review by a as a doctor, a clinician, a technologist, an operator, an expert, medical specialist, a technician, and/or the like unless the context indicates otherwise. It should be appreciated that the clinician may calibrate and/or set parameters of PAP devices operating in accordance with embodiments of the present system.

The terms patient, patients, and/or formatives thereof, may include patients or other individuals (e.g., persons, subjects, subjects-under-test, etc.) or other users who may be receiving any type of sleep apnea monitoring or assessment using systems, machines, processes, and/or methods operating in accordance with embodiments of the present system. It is assumed that the patients may have OSA and/or may have comorbidities. These comorbidities may include, for example, one or more of COPD, congestive heart failure (CHF), and/or the like. Generally, it will be assumed that when VST, such as PAP therapy or the like, is applied to a patient with OSA without comorbidities negative implications may include arousals and low sleep quality However, when VST is applied to a patient with OSA with comorbidities there may be additional cardiac implications as described herein.

Embodiments of the present system may be operative with ventilation support systems (VSTs) such as those described in a copending US patent application entitled "DETECTING, IDENTIFYING AND ALLEVIATING CAUSES FOR PRESSURE BURDEN USING POSITIVE AIRWAY PRESSURE DEVICES," having application number EP22214321.6, filed December 16, 2022.

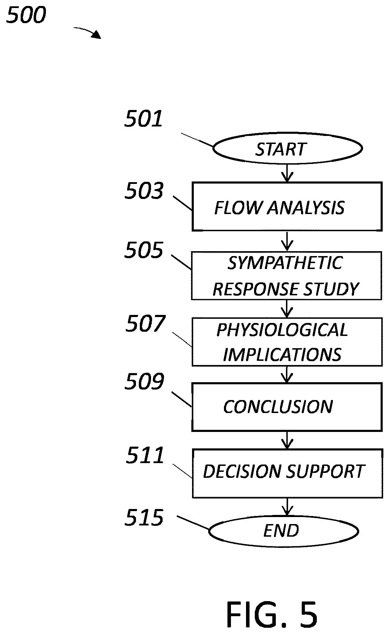

Embodiments of the present system may perform a sequence of data acquisition and data analysis to assist a decision support to adapt the PAP therapy to the patient. For example, in the following embodiments, the system may generally perform a sequence of data acquisition and data processing and decision support which may include acts of: (1) performing a flow analysis; (2) performing a sympathetic response study which may include an electrophysiological analysis, and performing a validation of this study to validate the determined sympathetic response is critical to trigger an arousal (of the patient); (3) determining physiological implications including hemodynamic alterations, and validation; (4) determining a conclusion; and (5) providing decision support to adapt PAP device settings. These steps or acts will be set forth below in more detail with reference to FIG. 5.

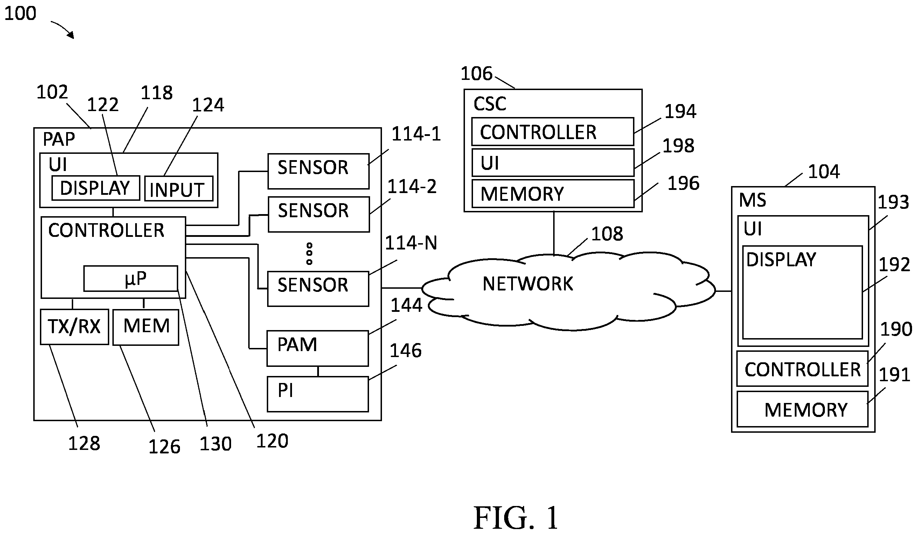

FIG. 1 illustratively shows a schematic block diagram of a portion of a system 100 (hereinafter the system 100) operating in accordance with embodiments of the present system. The system 100 may include one or more of a positive airway pressure (PAP) 102 device (hereafter PAP 102), a mobile station (MS) 104, and a clinical service center (CSC) 106, one or more of which may communicate with the other via a network 108, using any suitable communication method or methods such as wired, wireless, and/or optical communication methods. The PAP may provide VST to a corresponding patient.

The network 108 may include any suitable network such as an ad-hoc network, a Bluetooth^{™} network, a body area network (BAN), a personal area network (e.g., Bluetooth^{™}, etc.), a Zigbee network, Wi-Fi, a local area network (LAN), a wide area network (WAN), a telephony network, a cellular network (e.g., 5G, etc.), and/or combinations thereof. The PAP 102, the MS 104, the CSC 106, and the network 108 may be controlled by at least one controller which may control the overall operation of the system and may include one or more logic devices such as a microprocessor and/or the like.

The at least one controller may access at least one memory and may obtain system setting information (SSI) which may include one or more of operating parameters (e.g., scan type, initialization settings, etc.), operating settings, threshold values, warnings, display settings, user information, patient information such as patient identification (ID), content (e.g., video, audio, etc.), etc. The SSI may be set by the system and/or user and may be updated during use. For example, the SSI may be updated to reflect initial settings (e.g., default settings), etc., as they may be updated by the system and/or clinician. The at least one controller may generate and render an interface with which the patient and/or clinician may, for example, interact with to, for example, set, reset, select, and/or adjust one or more settings of the SSI and/or enter information (e.g., ID, test type, settings, parameters (e.g., test duration), results, etc.) which may be stored in the SSI in at least one memory of the system. The SSI may be different for each patient. The at least one controller may be a local and/or distributed throughout the system.

The MS 104 may include any suitable mobile station(s) such as a smart-phone (e.g., an I-Phone, a Galaxy phone, etc.), a personal digital assistant (PDA), a smart watch (e.g., an Apple^{™} Watch^{™}, a Galaxy^{™} watch, etc.), and/or the like, and may include a memory 191, at least one controller 190, a user interface (UI) 193, and a transceiver (TX/RX) configured to communicate with the network 108.

The UI 193 may provide an interface with which a user (e.g., the patient, the clinician, etc.) may interact, such interfaces may include, inter alia, a touch-screen display 192, an optical sensor such as a camera, a proximity sensor, a speaker, and/or a microphone. However, other user interfaces are also envisioned. The UI 193 may render information, such as content, etc., on the display and/or a speaker for the convenience of the patient or clinician, and may receive information entered by the patient or clinician such as selections, requests, commands, patient or clinician settings, etc., using any suitable input method such as gesture inputs, a touch input, voice input, etc. The memory 191, and/or other memories of the PAP 102 and/or the CSC 106, may store information for use by the system such as the SSI, sleep monitoring information (SMI), system settings, system parameters, operating parameters, and/or combinations thereof, as will be discussed below. The patient identification (ID) may be employed to identify the patient and the corresponding test results. The MS 104 may transmit information such as operating parameters, instructions, requests, commands (e.g., a synchronization command, etc.), to the PAP 102, and may receive information generated by the PAP 102 such as the SSI, operating parameters, sensor information, and/or combinations thereof, from the PAP 102 for further processing. The MS 104 may further receive and/or transmit sleep monitoring information (SMI), current settings, warnings, PAP status, etc., as discussed herein.

Data transfer may occur on a periodic or non-periodic intervals as may be set forth in the SSI and/or requested by the system (e.g., the PAP 102, the CSC 106, or the MS 104), patient, and/or clinician. For example, information may be transmitted from one or more of the PAP 102, the CSC 106, and the MS 104 to another of the PAP 102, the CSC 106, and the MS 104 (or internally within any of the PAP 102, the CSC 106, and the MS 104), in response to a query or request, and/or may be pushed at periodic or non-periodic intervals to a receiver depending upon system configuration and/or settings. Sensor information may be transferred directly or via, for example, the network 108, from one or more of the PAP 102, the CSC 106, and the MS 104 to another of the PAP 102, the CSC 106, and the MS 104, for further processing.

For the sake of clarity, only a single MS 104 will be discussed unless the context indicates otherwise. However, without limitation other MSs may be employed by the system. For example, a clinician such as a doctor may have a further MS in addition to the (primary) MS of the system 100, where one or more of the primary MS and the further MS may receive information such as SSI, SMI, etc., from other portions of the system for further review, processing, and/or storage. Accordingly, there may be a plurality of MSs 104 employed by the system 100 one or more of which may communicate with each other via the network 108 or any suitable communication link.

The CSC 106 may include one or more of a controller 194, a memory 196, and a UI 198. The controller 194 may control the overall operation of the CSC 106 and, as such may receive information, such as the SSI, SMI, and/or other information from the PAP 102 and/or MS 104 for further processing. The controller 194 may control operation of the system, if desired. However, it should be understood that any controller of the system may control operation of the system if desired, such as the controller 190 of the MS 104 and/or a controller 120 of the PAP 102. The CSC 106 may further communicate with the PAP 102 and/or MS 104 to, for example, transmit diagnostic results of the processing (e.g., operating mode(s), etc.) to be rendered on a rendering device of the system, such as on a rendering device of the MS 104, for the convenience of the user. The diagnostic results may be stored in the SMI in a memory of the system. The CSC 106 may process data in real time or may obtain data that has been stored in a memory of the system.

One or more controllers of the system 100 may employ machine learning and/or artificial intelligence (AI) engines to process information generated by the system such as sensor information, the SMI as well as data provided to the system such as historical information. The one or more controllers of the system 100 may be operative to monitor for and detect events, and form corresponding event information, etc., and/or may store and/or update results in a memory of the system (e.g., within the SMI), and may forward these results to the corresponding patient and/or clinician for their convenience as may be set forth, for example, in the SSI. The SMI may be accessed by one or more of the CSC 106, the MS 104, and the PAP 102 of the system for further review, processing, and/or analysis.

The PAP 102 may include one or more of a controller 120, a transceiver or transmitter/receiver (TX/RX) 128, a memory 126, sensors 114-1 through 114-N (generally sensors 114, where N is an integer), a pressure assist module (PAM) 144, a patient interface (PI) 146, and a user interface (UI) 118 with which a user, such as the patient or clinician, may interact. In accordance with embodiments, although N sensors 114 are illustratively shown, more or less (e.g., 1, 2) may be utilized in accordance with the present system. The controller 120 may control the overall operation of the PAP 102 and may communicate with one or more of the CSC 106, the MS 104, and the network 108, via the TX/RX 128. The controller 120 may include one or more logic devices such as a microprocessor (µP) 130 and may control the overall operation of the PAP 102. It should be appreciated that in some embodiments the controller 120 may include digital and/or analog control circuitry. It is further envisioned that the PAP 102 and/or portions thereof, such as one or more of the sensors 114 may be a stand-alone unit with its own power supply as well as with its own controller to control sensor operations and/or make determinations from sensor information measured by the sensor. Accordingly, optional power supplies and/or charging systems (e.g., wired, wireless, solar, etc.) may be provided to provide energy to the corresponding power supply depending upon system configuration. In some embodiments the one or more of the sensors 114 may include a sensor controller/processor (which may be part of and/or separate from the system controller 120) that controls sensor operations, as well as analyzes and makes determinations from the sensor information.

The UI 118 may include a display 122 (e.g., a touch-screen display) and a user input interface 124 which may be combined with, or separate from, each other. The user input interface 124 may include a keyboard, a mouse, a trackball, a touch-sensitive sensor, or other device, such as a touch-sensitive display, which may be stand alone or part of a system, such as part of a laptop, a personal digital assistant (PDA), a mobile phone (e.g., a smart phone), a smart watch, an e-reader, a monitor, a smart or dumb terminal or other device for communicating with the controller 120 via any operable link such as an optical, wired, and/or wireless communication link. The user input interface 124 may be operable for interacting with the controller 120 including enabling interaction within a UI 118 as described herein. Clearly the controller 120, the sensor 114, the user input interface 124, the user interface (UI) 118, the memory 126, the PAM 144, the PI 146, the CSC 106, the MS 104, and the network 108 may all or partly be a portion of a computer system or other device. The UI 118 may be operative to provide audio/visual feedback as well as graphical user interfaces (GUIs) to the user of the present system and may inform the operator of operating parameters, operating states, etc.

The controller 120 may be operative to render content, such as still or video information, as well as audio information on a rendering device of the system such as on the display 122 and/or speaker of the UI 118. This information may include information related to operating parameters, instructions, feedback, events, and/or other information related to an operation of the system, or portions thereof, such as SSI or portions thereof, SMI, application data, data generated by the system, operating parameters, etc., and/or combinations thereof. Clinical decision support (CDS) may be employed to analyze data generated by the system and may generate determinations (e.g., clinical decisions (CDs)) which may be stored in the system and/or rendered for the convenience of the patient and/or clinician depending upon settings in the SSI. For example, the CDs may be stored in the SMI and/or the SSI, where the SSI may include system setting information that may be used by the system so set operational parameters and settings as may be set by the system and/or user. The SSI may be updated in real time to reflect, for example, current settings and/or operational parameters of the system, depending upon system settings.

The controller 120 may be operable for providing control signals and/or performing operations in response to input signals from the user input device 124 as well as in response to other devices of a network, such as the sensor 114, and executing instructions stored in a memory of the system such as the memory 126. The µP 130 may include one or more of a microprocessor, an application-specific and/or general-use integrated circuit(s), a logic device, etc. Further, the µP 130 may be a dedicated processor for performing in accordance with the present system and/or may be a general-purpose processor wherein only one of many functions operates for performing in accordance with the present system. The µP 130 may operate utilizing a program portion, multiple program segments, and/or may be a hardware device utilizing a dedicated or multi-purpose integrated circuit. It is envisioned that one or more portions of the PAP 102, such as the controller 120, may be operationally coupled to the memory 126, the TX/RX 128, the user interface (UI) 118, the rendering device such as a display 122, the sensor 114, the user input interface 124, the PAM 144, the PI 146, and the network 108.

The memory 126 include any suitable memory configured to store information used by, or generated by, the PAP 102 such as operating instructions, the SSI, the SMI, etc. In some embodiments, the memory 126 may store information related to operating conditions, such as operating modes, as well as PAP control parameters (e.g., EPAPmax, PSmax, Pmax, etc.) of the PAP 102. The modes may include one or more of CPAP, BiPAP, APAP, ASV, and/or the like. The settings may include one or more of C-flex, Bi-flex, A-flex, and/or the like. The control parameters may include one or more of pressure, flow, time-based parameters, etc. The time-based parameters may include one or more of respiration rate variability, timely flow changes in transition from inspiration to expiration and vice versa, peak flow, flow limitations, and/or the like.

The memory 126 may be any type of device for storing application data as well as other data related to the described operation such as application data, data generated by the system, operating parameters, SSI, SMI, etc., and/or combinations thereof. The application data, data generated by the system, operating parameters, SSI, SMI, etc., and/or combinations thereof, may be received by the controller 120 for configuring (e.g., programming) the controller 120 to perform operation acts in accordance with embodiments of the present system. The controller 120 so configured becomes a special purpose machine particularly suited for performing in accordance with embodiments of the present system.

The methods of the present system are particularly suited to be carried out by a computer software program, such program containing modules corresponding to one or more of the individual steps or acts described and/or envisioned by the present system. Such program may of course be embodied in a computer-readable medium, such as an integrated chip, a peripheral device or memory, such as the memory 126 or other memory coupled to the controller 120.

The program and/or program portions contained in the memory 126 may configure the controller 120 to implement the methods, operational acts, and functions disclosed herein. The memories may be distributed, for example between the clients and/or servers, or local, and the controller 120, where additional processors may be provided, may also be distributed or may be singular. The memories may be implemented as electrical, magnetic, or optical memory, or any combination of these or other types of storage devices. Moreover, the term "memory" should be construed broadly enough to encompass any information able to be read from or written to an address in an addressable space accessible by the µP 130 of the controller 120. With this definition, information accessible through a network such as the network 108 is still within the memory, for instance, because the µP 130 may retrieve the information from the network 108 for operation in accordance with embodiments of the present system.

The sensor 114 may be situated at one or more portions of the system and may sense related parameters, form corresponding sensor information, and provide this sensor information to the controller 120 and/or to its own controller for further processing. For example, the sensor 114 may include sensors which may be specific to a test type being performed. The sensor 114 may be distributed throughout the system 100 and may include one or more sensors which may communicate with each other and/or other portions of the system such as the controller 120, the TX/RX 128, etc., using any suitable wired, wireless, and/or optical communication method or methods. It is also envisioned that in some embodiments one or more of the sensors 114 may provide sensor information continuously, in response to a query, and/or on at periodic and/or non-periodic intervals. For example, in some embodiments, one or more of the sensors 114 may push data at periodic and/or non-periodic intervals to the controller 120, depending upon system settings as may be set forth in the SSI. In yet other embodiments, it is envisioned that one or more of the sensor 114 may provide data to the controller 120 in response to a query. In some embodiments, one or more of the sensor 114 may transmit bursts of sensor information to the controller 120 at periodic intervals (e.g., every 5 minutes, etc.) to save power by transmitting in burst at periodic intervals rather than transmitting continuously, the latter of which may drain power from a power source especially when operating as a remote wireless sensor 114 such as a remote biosensor. In accordance with embodiments of the present system, the sensor 114 may include any suitable sensors or sensors such as wearable body worn sensors, remote sensors, sensors coupled to CPAP device and/or sensors coupled to the patient interface (PI) 146.

The controller 120 may be configured to communicate with the MS 104 using any suitable communication method or methods via, for example, the network 108. The controller 120 may obtain the SSI from the MS 104 and store this information in the memory 126 for reference at a later time and/or to configure its operation in accordance with the SSI. During operation, the controller 120 may receive analog signals from the sensor 114, amplify (e.g., using one or more amplifiers) and/or condition these analog signals (e.g., using one or more signal conditioners), convert these signals (e.g., using one or more analog-to-digital (A/D) converters) to digital signals (e.g., at a desired sampling rate (as may be set in the SSI)) to form corresponding information which may be further processed or may be stored in the SMI for later use and/or processing. In some embodiments, it is envisioned that one or more of the sensors 114 may process signals as discussed above, such as via one or more sensor controllers of the one or more of the sensors 114 and transmit corresponding digital signals to the controller 120 for further processing. It is further envisioned that in some embodiments, one or more of the sensors 114 may be wirelessly paired to the controller 120. It is also envisioned that one or more of the sensor 114 may be remotely located relative to the controller 120 of the PAP 102. Accordingly, the remote sensor 114 may communicate with the controller 120 via the TX/RX 128 using any suitable wireless communication method or methods which, without limitation, may include Bluetooth^{™}, WiFi^{™}, etc.

The PAM 144 may include one or more an air pump(s), blowers, pressure regulator(s), flow regulator(s), flow valve(s), and flow monitor(s) and may be configured to provide airflow at a desired pressure and/or flow (e.g., volume) to the patient under the control of the controller 120. The PAM 144 may be coupled to the patient via the patient interface (PI) 146. The PAM 144 may provide an output in accordance with one or more operational modes, such as a multimodal continuous positive airway pressure (CPAP) mode, Automatic-CPAP (APAP), Automatic/Adaptive Servo Ventilation (ASV) mode, Average volume assured pressure support (AVAPS), Bilevel mode and/or other modes, under the control of the controller 120. For simplicity, a CPAP is illustratively described and referred to herein, however, in accordance with embodiments, other PAP devices may be suitably utilized.

The patient interface (PI) 146 may include any suitable patient interface that may deliver a flow of air to the patient such as a mask, a nasal canula, and/or the like. The patient interface (PI) 146 may be coupled to the PAM 144 via a line-set and may include one or more sensor 114 of the sensors 114-N, such as a capnograph including a CO2 sensor, one or more flow sensors, etc. depending upon system configuration.

The controller 120 may monitor the sensors 114 and process data to enable pressure management decisions in accordance with embodiments of the present system that may ensure that cardiac output is preserved and/or excessive sympathetic activation is not generated. The controller 120 may analyze sensor information to detect sympathetic activation and may be configured to realize these pressure management decisions by controlling the PAM 144 to be configured to provide flow according to a desired operational mode such as a continuous positive airway pressure (CPAP) mode where the same amount of air pressure is provided for both inhalation and exhalation, Automatic/Adaptive Servo Ventilation (ASV) mode, automatic PAP (APAP) mode, bilevel PAP (BIPAP) mode where the different amount of air pressure is provided for inhalation and exhalation, APAP, or other modes as may be desired. In ASV mode, positive pressure may be provided when apneas or hypoxias are detected. In accordance with embodiments of the present system when in ASV mode the system may sense the ventilation needs of the patient and may adjust support accordingly to, for example, provide little support (pressure) when normal breathing is detected, and more support when abnormal breathing is detected. However, other modes are also envisioned. The controller 114 is illustratively described as performing various steps, acts or operations such as analyzing, etc., to simplify the discussion herein. However, in accordance with embodiments, one or more other controllers may perform one or more of such operations. Accordingly, the discussion herein regarding the controller 120 should be understood to similarly apply to any one or more of these other controllers.

One or more of the sensors 114 may include sensors of one or more types such as pressure sensors, flow sensors, temperature sensors, carbon dioxide sensors (CO2), optical sensors, bioimpedance sensors, electrical impedance tomography (EIT) sensors, electrocardiogram (ECG) sensors, electromyogram (EMG), electroencephalogram (EEG), electrooculography (EOG, eye movement), photoplethysmogram (PPG) sensors, galvanic skin response (GSR) sensors, balistocardiogram (BCG) sensors, optical sensors (e.g., to determine peripheral oxygen concentration (SpO2) using pulse oximetry and/or to determine heart rate), chest band sensors (e.g., to sense chest expansion and/or contraction), microphones (e.g., to detect snoring, etc.), accelerometers (e.g., for body movement and/or body position), and/or the like that may be paired to the controller 120 via wired, wireless, and/or optical communication methods and may provide corresponding sensor information thereto. The chest band sensors may include strain sensors (to detect movement, strain, etc.), accelerometers (e.g., to detect movement, acceleration, etc.), EIT sensors, EMG sensors (e.g., to detect muscle activity) and/or position sensors which may be integrated with or otherwise coupled to, the chest band sensors. The chest band sensors may be coupled to, or formed integrally with, a belt, a strap, etc., (hereinafter generally belt) and may sense, for example, movement, acceleration, position, and/or muscle activity of the patient.

In some embodiments, the controller 120 may employ one or more algorithms described herein to monitor the patient's risk factors for poor clinical outcomes and command changes in the flow settings to optimize overall patient outcomes by controlling the PAP 102 accordingly to provide the proper pressure and flow to the patient coupled to PAP 102 via the PI 146. This may provide for proper pressure management decisions that may ensure that cardiac output is preserved, hyperinflation (e.g., beyond a threshold) is avoided, and excessive sympathetic activation is not generated in the patient. In accordance with some embodiments, sensory modalities may include one or more sensors which can provide sensory information that may enable the controller 120 to noninvasively monitor cardiac and pulmonary phenomena and control the PAM 144 accordingly to output an airflow at a desired pressure and flow. The system may include a capnograph having a CO2 sensor that may sense CO2 concentration in expired air to determine (estimated) gas trapping in the lung (alveoli) and form corresponding sensor information that may be provided to the controller for further processing and storage. The chest band sensors may measure extension of the chest. Two band sensors may be employed one on the chest of the user/subject and the other on the abdominal area to measure breathing pattern and extension of the chest. However, it is also envisioned that extension of the chest may be detected by optical sensors that may perform image analysis to determine extension of the chest. The optical sensor may be considered an external sensor and may detected body position and/or movements of the patient, form corresponding sensor information, and provide this sensor information to the controller for further processing and/or analysis. The band sensors may be situated at the chest or abdomen of the patient and may include a strap or belt including one or more strain sensors or the like which may sense a corresponding extension of the chest or abdomen of the patient, form corresponding sensor information, and provide this sensor information to the controller for further processing and/or storage. Accordingly, the controller may determine extension, contraction, and/or movement of the corresponding chest or abdomen of the patient. Sympathetic activity can be measured using one or more methods such as through analysis of one or more of heart rate variability (HRV), blood pressure, the breathing pattern, the galvanic skin response (GSR), brain waves (EEG), limb, head, and/or body movements, facial expressions, and/or the like. Sensor types may include, for example, optical (e.g., PPG, camera), acceleration (e.g., accelerometer), bioimpedance, flow, chemical (e.g., CO2 which may indicate hyperventilation) and/or electrical (electrodes) sensors. In accordance with some embodiments, one or more of the sensors may be integrated with, or otherwise coupled to, a face mask, a body of the system, a skin-contacting wearable, and/or a camera. With regard to sympathetic activity, it is envisioned that this may be measured using one or more methods, such as by analyzing information indicative of heart rate variability (HRV), blood pressure, breathing pattern, galvanic skin response (GSR), brain waves (e.g., EEG), movement information (e.g., limb, head, and/or body movement), facial expression(s), etc. to determine sympathetic activity. The one or more sensors may include one or more external sensors (e.g., a remote biosensor such as a camera which may be configured to sense body position and/or movement of the patient). With regard to respiration drive, this can be assessed using any suitable method or methods such as via analysis of diaphragmatic electrical activity (electrodes), diaphragmatic ultrasound (muscle activity), sensing muscle activity of other muscles which are involved in active expiration (abdominal, intercostal), e.g., through analysis of EMG information. It is also envisioned that respiration drive may be assessed through flow pattern analysis (e.g., risetime, overshoot in transition of inspiration to expiration and vice versa).

The PAP 102 may include any suitable measurement system(s) to monitor the physiological parameters of the patient/subject/user as they change over time during use using one or more methods to determine changes in physiological parameters of the patient from which physiological features of the patient may be derived. These measurement system(s) may have process flows and operations that may be stored in the SSI and may be selected by the SSI, the patient, and/or the clinician as may be desired. In accordance with embodiments of the present system, it is envisioned that the PAP 102 may perform a training process in which the system may monitor the patient's physiology during use using one or more operating modes, form corresponding sensor information, process the sensor information in accordance with embodiments of the present system, determine operating parameters, operating modes, pressures, flow rates, etc., and thereafter store the results in the SMI for later use such as when critical hyperinflation and/or excessive sympathetic activity is detected by the sensors and/or controller. The operational modes may be selected by the system (e.g., automatically) based upon patient types and/or comorbidities, or may be selected by the clinician or patient.

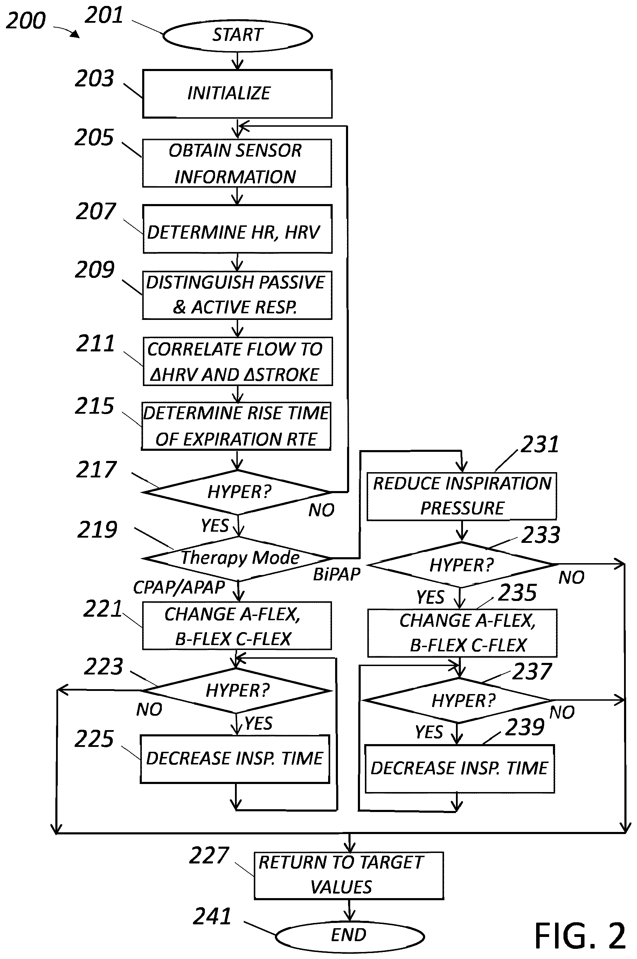

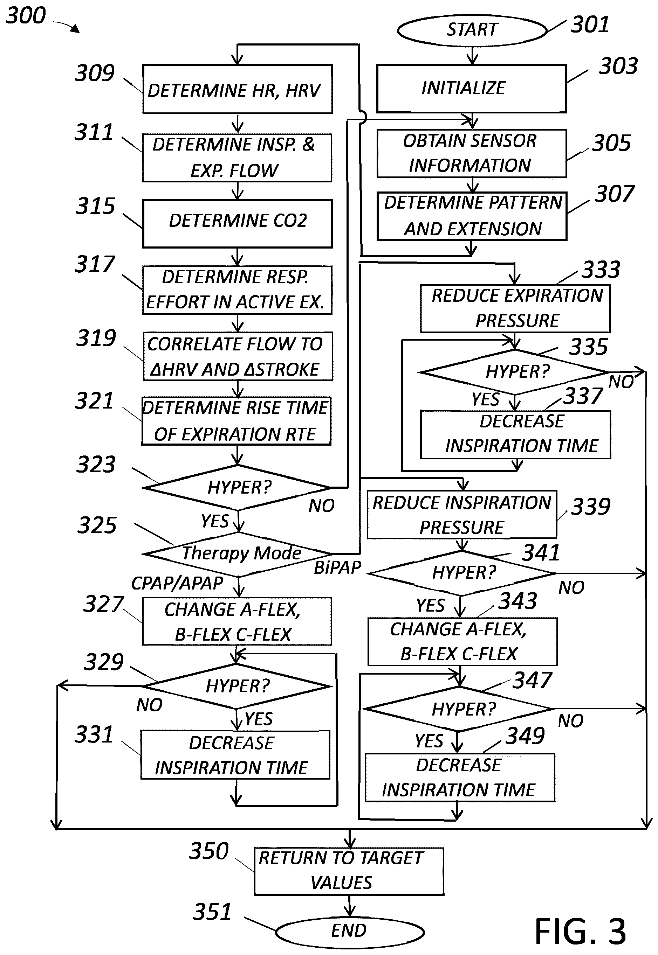

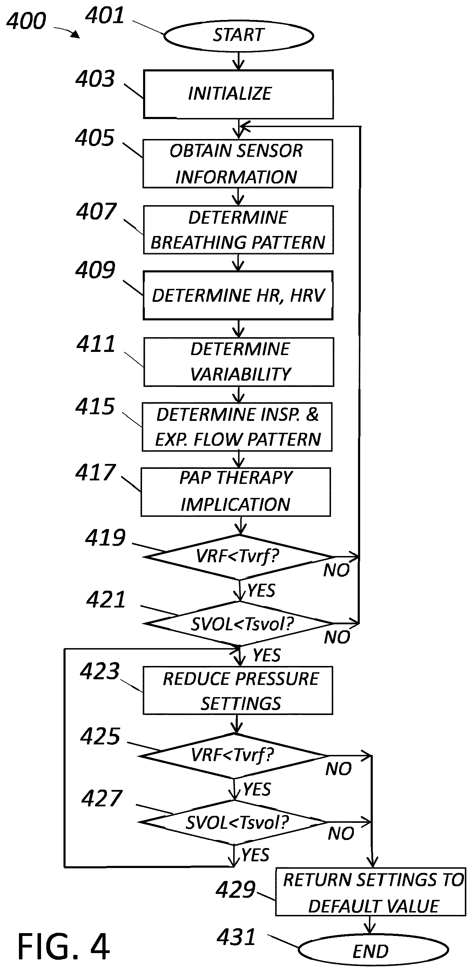

Operational processes performed by the system will now be described with reference to FIGs. 2 through 5 where FIG. 2 illustratively shows a functional flow diagram performed by a process 200 performed for a patient with sleep-disordered breathing (SDB) patient in accordance with embodiments of the present system; FIG. 3 illustratively shows a functional flow diagram performed by a process 300 for chronic obstructive pulmonary disease (COPD) and obstructive sleep apnea (OSA) comorbid patients in accordance with embodiments of the present system; FIG. 4 illustratively shows a functional flow diagram performed by a process 400 for resolving diminished cardiac output in response to ASV therapy with SDB patients comorbid with CHF in accordance with embodiments of the present system; and FIG. 5 illustratively shows a functional flow diagram performed by a process 500 for assisting decision support to adapt PAP therapy accordance with embodiments of the present system.

The processes 200, 300, 400, 500, may be performed using one or more processors, computers, controllers, etc., communicating over a network and may obtain information from, and/or store information to, one or more memories which may be local and/or remote from each other. The processes 200, 300, 400, 500 may include one of more of the following acts. In accordance with embodiments of the present system, the acts of the processes 200, 300, 400, 500 may be performed using a controller operating in accordance with embodiments of the present system. Further, one or more of these acts may be combined and/or separated into sub-acts, or performed serially or in parallel, as may be desired. Further, one or more of these acts may be skipped depending upon settings. For the sake of clarity, the process may be described with reference to a single PAP and a single patient case. However, without limitation, it should be understood that the process may employ a plurality of PAPs and a plurality of patient cases each of which may include a separate workflow such as a sub-workflow.

With reference to FIG. 2, in operation, the process 200 may start during act 201 and then proceed to act 203 where it may initialize the PAP device 102 by performing an initialization routine. During initialization, the process may load SSI and may set system settings in accordance with the SSI. The SSI may further include information related to operation or therapeutic modes (e.g., continuous PAP (CPAP), automatic PAP (APAP), bilevel PAP (BIPAP), AutoCPAP modes, etc.), target values, thresholds, initial or default modes, process flow, sensor parameters, and/or other settings. The PAP control parameters may be defined in the SSI and may be modified by the system and stored as for later use. During the initialization, one or more sensors (which may include one or more external sensors) may be paired to the controller (e.g., controller 120), automatically, or by the clinician and/or the patient. Accordingly, during operation, one or more of the sensors may communicate with the controller via a wireless communication link such as Bluetooth^{™}, WiFi^{™}, and/or the like. The process may also begin an application interface (API) configured to enable wired and/or wireless communication between the controller and the one or more sensors. It will be assumed that the process may start in a default (e.g., an initial) mode of operation such as normal, Bi-pap, C-flex, etc., and may be operative initially in accordance with this default mode. Thereafter, depending upon sensed parameters and/or selection by a clinician (e.g., as may occur in response to a complaint (e.g., discomfort, poor sleep quality, etc.) from the patient, etc.), the system may switch into a hyperventilation (intervention) mode automatically or in response to the clinician's request, respectively, to perform one or more hyperventilation intervention operations which may reduce or entirely eliminate the hyperventilation. Thus, when the system, for example, detects hyperinflation in combination with active expiration followed by arousals, the system may switch into a hyperventilation mode. The hyperventilation (sensing or intervention) mode may be performed using a system which may have the capability to vary inspiration and expiration pressure during therapy such as Bi-Pap, C-flex, etc.

After completing act 203, the process may continue to act 205 during which the process may obtain sensor information from one or more sensors of the system such as from chest and abdominal sensors (e.g., from the chest band sensors) which may measure breathing pattern and extension of the chest and form corresponding sensor information, that may be transmitted to a controller of the system for further processing. Similarly, one or more electrocardiography (ECG) sensors may sense physiological parameters of a chest of the patient and form corresponding ECG information that may be provided to the controller for further processing. One or more flow sensors may monitor inspiratory and expiratory flow (patterns) to, or from, the patient, respectively, and form corresponding flow information that may be provided to the controller of the system. PPG sensors may measure variability in stroke volume (SV), ejection fraction (EF) and/or cardiac output (CO). The sensor information may be collected by the controller continuously in real time or at periodic or non-periodic intervals.

After completing act 205, the process may continue to act 207 during which the process may analyze the ECG sensor information to determine one or more of heart rate (HR) and heart rate variability (HRV) the latter of which is a variation in the time interval between heartbeats (aka, cycle-length variability, R-R variability, and heart period variability. The process may further analyze the flow information to determine inspiratory and expiratory flow patterns. The process may analyze the change of HR/HRV and one or more hemodynamic parameters (e.g., a physiological parameter) are linked to a change of sympathetic activity caused by hyperinflation. The flow patterns may then be analyzed with other sensor information as discussed during act 207 to distinguish active and passive expiration. Thus, when hyperinflation is detected via any suitable method(s) such as via analysis of flow, and is classified as problematic (e.g., above threshold (e.g., in terms of time or chest expansion), in combination with active expiration, followed by arousals sensed with ECG, followed by a decreased cardiac output/stroke volume/ejection fraction, and/or increased CO2), a hyperinflation intervention mode may be activated to control the critical hyperinflation.

After completing act 207, the process may continue to act 209 during which the process may distinguish passive and active respiration. Accordingly, the controller may process the flow information and distinguish between passive and active expiration using any suitable method or methods. For example, the passive expiration may be determined when the controller detects an expiratory flow (e.g., an outflow) with respiratory activity (RA) that is determined to be less than or equal to a respiratory activity threshold (TRA) (e.g., 0 in the present embodiments although other values are also envisioned). Thus, passive expiration may be detected during expiration when it is detected that RA is less than or equal to TRA which occurs when there is little to no respiratory muscle activity; and active expiration may be determined when the controller detects that RA is greater than TRA during expiration when there is excessive (e.g., greater than the respiratory activity threshold) respiratory muscle activity which also may be detected by the controller in conjunction with muscle activity sensors, e.g.,, accelerometers, EIT sensors, EMG sensors and/or chest and/or abdominal sensors. Thus, the process may obtain flow information and perform a timely analysis of it to detect one or more of passive and active expiration and may distinguish these from each other. It should be appreciated that increased hyperinflation leads to a forced outflow of air in the passive expiration cycle. This requires increase muscular effort on the chest muscles of the patient and may be detected, for example, through analysis of muscular effort determined through analysis of EIT and/or EMG sensor information. For example, the process may analyze EMG sensor information (e.g., to determine measurement of the muscular effort) and the EIT sensor information to complement the measurement of the muscular effort by adding volumetric data (e.g., which may be indicative of chest inflation) caused by the muscle activity.

After completing act 209, the process may continue to act 211 during which the process may analyze cardiac data included in the ECG sensor information or other suitable cardiac sensor(s), such as PPG for example, and determine a change in cardiac performance, such as cardiac output, ejection fraction and/or stroke volume. This change is then correlated to the changes in the HRV and stroke volume (SV) as a surrogate for increased sympathetic activity. These changes to the HRV and SV may be referred to as (ΔHRV) and (ΔSV), respectively. The stroke volume (SV) is a volume of blood pumped from the left ventricle per beat. The change in cardiac performance may be detected within one or a few heart cycles, hyperinflation changes the venous return flow and the cardiac preload/afterload. Sensors (in heart and central vascular structures) may sense it immediately the sympathetic outflow is a direct consequence.

After completing act 211, the process may continue to act 215 during which the process may, in the time domain, determine the rise time of expiration (RTE). Accordingly, the process may analyze, in the time domain, the flow information from the flow sensor(s) to determine the rise time of the expiration (RTE). The rise time of expiration (RTE) indicates how fast the patient exhales. RTE in rest (e.g., during passive expiration) may be employed as a reference, where a change in RTE is indicative of an active expiration, and a quantified analysis may then be related to the effort in breathing. The process may employ an algorithm (e.g., a hyperinflation detection algorithm (HDA)) to determine a level of hyperinflation (in combination with chest sensors) and this information may then be employed as a decision support to adapt the PAP device settings.

After completing act 215, the process may continue to act 217 during which the process may determine whether critical hyperinflation is detected. Accordingly, if it is determined that critical hyperinflation is detected, the process may continue to act 219. However, if it is determined that critical hyperinflation is not detected, the process may return to act 205 and repeat acts 205 to 217. To determine whether critical hyperinflation is detected (e.g., which may be considered a critical state), the process (via, for example, the hyperinflation detection algorithm) may compare the RTE with a threshold rise time (Trte) or may employ any other suitable method(s) as discussed in this application. Accordingly, when it is determined that the RTE is less than the Trte, the process may determine that critical hyperinflation is detected. However, when the process determines that RTE is not less than the Trte (i.e., RTE is greater than or equal to Trte), the process may determine that critical hyperinflation is not detected. When the RTE is less than the threshold rise-time (Trte) indicating critical hyperinflation, it is assumed a further shortening of the rise time will trigger an arousal (of the patient). In other words, any further shortening of the rise time (RTE) may arouse the patient which is may not be desirable. With regard to the rise time, the rise time may be a surrogate for sympathetic activity and the probability of a triggered arousal. The shortening of the rise time is the response of a sympathetic activity (change), when the sympathetic activity is above a certain value (e.g., a trigger threshold for an arousal) an arousal can be triggered. This value can be used to predict the likelihood of an arousal. Embodiments of the present system may adapt PAP settings to mitigate a sympathetic activity response, avoiding a value that is above the trigger threshold for an arousal. The threshold rise time (Trte) may be predetermined (and saved in a memory of the system), such as based on physiological condition of current subject and/or average values obtained experimentally from a population of subjects similarly situated to the current subject, and obtained from the memory of the system such as from the SSI. It is envisioned that the memory may be dynamically updated and by self-learning algorithms the settings of the PAP machine may be adapted to optimize the PAP therapy. Thus, the process may determine that hyperinflation is critical if it is determined that RTE is less than the threshold rise time (Trte). Conversely, the process may determine that hyperinflation is not critical if it is determined that RTE is not less than (e.g., is greater than or equal to) Trte.

With respect to hyperinflation, as hyperinflation reaches a critical level, pressure on the chest increases with the increase in inflation which may activate baroreceptors and stretch receptors, which may lead to an elevated sympathetic outflow (e.g., sympathetic nervous system activity). Unfortunately, this increased sympathetic activity may be a cause for arousals, perceived discomfort, and non-adherence to CPAP therapy and the process may be configured to avoid this. Accordingly, embodiments of the present system may monitor chest inflation and take actions such as enter a hyperinflation intervention mode or the like by controlling a ventilation support therapy (VST) device such as a PAP machine (e.g., by controlling device settings) in accordance with embodiments of the present system to reduce chest inflation and/or reduce the sympathetic outflow to enhance user comfort during use of a positive air pressure device such as a CPAP, APAP, BiPAP, etc.

During act 219, the process may determine a current therapy mode. Accordingly, if the current mode is CPAP or APAP mode the process may continue to act 221. However, if it is determined that the current mode is BiPAP, the process may continue to act 231. Although, APAP, CPAP, and BiPAP modes are illustrated for the sake of clarity in the current embodiments, other therapy modes are also envisioned and may have process flows defined for them.

During act 221, the process may change the C-flex, B-flex, and/or A-flex settings (also referred to as Cflex, Bflex and Aflex) to corresponding Cflex, Bflex, and Aflex threshold values, respectively, to ease the outflow of air during expiration. The C-flex, B-flex, and/or A-flex settings provide a comfort feature by relieving pressure during inhalation, exhalation and/or transitions from inhalation to exhalation during therapy. For example, the process may, depending upon system settings, decrement the Aflex settings, by, for example, a desired Aflex decrement value (Adec) (as may be set forth in the SSI) of one, although other values are also envisioned. Thus, a current Aflex setting may be decremented by Adec such that (Aflex (next) = Aflex (current) - Adec). Similarly, the process may, depending upon system settings, decrement the Bflex settings, by, for example, a desired Bflex decrement value (Bdec) (as may be set forth in the SSI) of one, although other values are also envisioned. Thus, a current Bflex setting may be decremented by Bdec (e.g., which is a decrement step size) such that (Bflex (next) = Bflex (current) - Bdec). Similarly, the process may, depending upon system settings, decrement the Cflex settings, by, for example, a desired Cflex decrement value (Cdec) (as may be set forth in the SSI) of one, although other values are also envisioned. Thus, a current Cflex setting may be decremented by Cdec (e.g., which is a decrement step size) such that (Cflex (next) = Cflex (current) - Cdec). Accordingly, when changed, C-flex, B-flex, and A-flex pressures may be reduced by one in the present example. However, other values of Adec (e.g., Adec = 0.1 Aflex(current), or 0.2Aflex(current, or 0.5Aflex(current.), Bdec, and/or Cdec (e.g., which is a decrement step size are also envisioned) which may increase the granularity of the corresponding one of Adec, Bdec, and Cdec. In some embodiments, there may be eight step sizes.

It is also envisioned that the settings of C-flex, B-flex, and/or A-flex may be set to corresponding specified predetermined threshold values. For example, the C-flex setting be set to a Cflex threshold value (Ct) which may correspond to a desired pressure drop that may reduce the air pressure supplied by the system during each exhale (e.g., expiration) by the patient. The Cflex threshold value (Ct) may be an integer, such as two, in the present embodiments although over values are also envisioned and may be set forth in the SSI. With regard to the B-flex setting, this setting may be set to a Bflex threshold value (Bt) which may correspond to a desired pressure drop that may reduce the air pressure supplied by the system during each exhale (e.g., expiration) by the patient. The Bflex threshold value (Bt) may be an integer, such as two, in the present embodiments although over values are also envisioned and may be set forth in the SSI. With regard to the A-flex setting, this may be set to the Aflex threshold value (At) which may correspond to a pressure drop that may reduce the air pressure supplied by the system during each inhale (e.g., inhalation) by the patient. The Aflex threshold value may be an integer, such as two, in the present embodiments although over values are also envisioned and may be set forth in the SSI. For example, the Aflex threshold value (At) may be set to provide eight steps of pressure drops.

After completing act 221, the process may continue to act 223 during which the process may determine whether hyperinflation is critical. Accordingly, if it is determined that hyperinflation is critical, the process may continue to act 225. However, if it is determined that hyperinflation is not critical, the process may continue to act 227. The process may determine that hyperinflation is critical using any suitable method such as determining if the RTE is smaller than the threshold rise time (Trte) as discussed above with respect to act 217. The level of inflation correlates to the strength of the passive recoiling of the chest and diaphragm. This recoiling is a driving force in expiration. The shorter the rise time (RTE) the stronger the hyperinflation. Thus, as RTE decreases, hyperinflation increases.

During act 225, the process may shorten the time period for the pressure support during inspiration to decrease the tidal volume for M consecutive breath cycles, where M is an integer and may be set to 10 in the current embodiments, for example. However, other values for M are also envisioned and may be set forth in the SSI. Thus, the inspiration time interval may be shortened which is a time interval during which pressure support may be provided during inspiration. Thus, this time interval may be assumed to be the time period for pressure support during inspiration. The inspiration cycle could be longer than the pressure support interval. After completing act 225, the process may repeat act 223. Accordingly, hyperinflation may be monitored, and the above protocol may be continued until the target for a decreased chest inflation is reached and the controller 120 determines that hyperinflation is not or (no longer) critical.

During act 227, the process may return to target values as were initially set before the current process detected that hyperinflation is critical. The target values may be stored in a memory (e.g., memory 126) of the system during the initialization (e.g., act 203) and/or may be obtained from the SSI (which may be set the system as default setting, and/or set by the operator and/or patient and also stored in the system memory). Accordingly, the process may access the memory and/or the SSI for these default values. The process may also store current values for one or more settings of the system such as pressure and duration (e.g., inspiratory pressure and duration as well as expiratory pressure and duration, values for A-flex, Bi-flex (hereinafter B-flex), C-flex, etc.) e.g., in the SMI within a memory of the system such that if critical hyperinflation is detected again, the process may (look up the SMI) and return to the values that eliminated or reduced the critical hyperinflation (e.g., as first detected during act 217). In this regard, embodiments of the present system may provide sensing systems for hyperinflation, arousal, and airway pressure/resistance, and a controller with a learning and feedback loop for optimal (personalized) pressure adaptation, to improve the apnea hypopnea index (AHI) and sleep quality of the patient. Then, upon or in response to detecting critical hyperinflation, the system may immediately switch to the previous values that were found to eliminate or reduce the critical hyperinflation. Thus, the process may learn these values and store them in a memory of the system such as in the SMI for later use. With regard to A-flex, this may be considered a subset of B-flex. A-flex may be operative on APAP devices and B-flex may be operative on BiPAP devices. After completing act 227, the process may continue to act 241 where it may end.

During act 231, the process may reduce inspiration pressure by a threshold pressure value (J) (where J is an integer) for the N (where N is an integer and is equal to 5 in the current embodiments although other numbers are also envisioned) next inspiration cycles to decrease tidal volume and to reduce the hyperinflation. It is known that the effect of positive pressure on hyperinflation can be the opposite where increased positive pressure reduces hyperinflation. For example, in some cases PAP may contribute to hyperinflation, while in patients with COPD, PAP may be operative to keep the bronchial system and airways open which enables lung deflation. In the latter case, it is beneficial to solve hyperinflation via the inspiration phase. Hyperinflation may occur during PAP therapy. In COPD patients (even without PAP therapy) patients tend to hyperinflate to create an intrinsic PEEP to keep the central airways open (bronchi). Often patients tend to hyperinflate stronger than needed. When under a PAP therapy, patient-initiated hyperinflation is no longer needed in patients with COPD. Accordingly, the PAP machine may take control and can more readily adapt the pressure to the needs of the patient and may lower the level of hyperinflation. For example, at greater than or equal to 10 mm H₂O, the lung may be considered to be deflated compared to no PAP. Accordingly, a controller may be operative to control a blower or pump of the system such as the PAM 144, (and/or control the PAM pressure and/or flow, and/or control regulators and/or valves, etc.) to reduce inspiratory positive airway pressure (IPAP) by an amount as set forth by a threshold pressure value J.

After completing act 231, the process may continue to act 233 during which the process may determine whether hyperinflation is critical. Accordingly, if it is determined that hyperinflation is critical, the process may continue to act 235. However, if it is determined that hyperinflation is not critical, the process may continue to act 227. The process may determine that hyperinflation is critical if the RTE is less than the threshold rise time (Trte) as discussed above with respect to act 217. Thus, if the decline of the inspiratory BiPAP pressure performed during act 231 is insufficient, the pressure protocol as described for the APAP device may be employed.

During act 235, the process may change the C-flex, B-flex, and/or A-flex settings to corresponding Cflex, B-flex, and Aflex thresholds values, respectively, to ease the outflow of air during expiration. For example, the process may, depending upon system settings, decrement the Aflex settings, by, for example, a desired Aflex decrement value (Adec) of one (in the current embodiments, although other values are also envisioned, as may be set forth in the SSI). This act may be similar to act 221. Accordingly, reference is made to act 221 for a further description thereof.

After completing act 235, the process may continue to act 237 during which the process may determine whether hyperinflation is critical. Accordingly, if it is determined that hyperinflation is critical, the process may continue to act 239. However, if it is determined that hyperinflation is not critical, the process may continue to act 227. The process may determine that hyperinflation is critical if the RTE is less than the threshold rise time (Trte) as discussed above with respect to act 217.

During act 239, the process may reduce inspiration pressure by a threshold pressure value (J) (where J is an integer) for the N (where N is an integer and is equal to 5 in the current embodiments although other numbers are also envisioned) next inspiration cycles to decrease tidal volume and to reduce the hyperinflation. Due to the reduced inspiration pressure, less air is blown in, and the lower expiration pressure eases the expiratory outflow, consequently lowering the level of hyperinflation. Accordingly, a controller of the system may be operative to control the PAM, to reduce inspiratory positive airway pressure (IPAP) by an amount as set forth by a threshold pressure value J. This act 239 may be similar to act 225. After completing act 239, the process may repeat act 237 for the next inspiration cycles. Accordingly, hyperinflation may be monitored, and the above protocol may be continued until the target for a decreased chest inflation is reached.

With reference to FIG. 3, in operation, the process 300 may start during act 301 and then proceed to act 303 where it may initialize the PAP device 102, etc., by performing an initialization routine. During initialization, the process may load SSI and may set system settings in accordance with the SSI. As this act may be similar to act 203. Accordingly, reference is made to act 203 for further description of this act.

After completing act 303, the process may continue to act 305 during which the process may obtain sensor information from one or more sensors of the system such as from chest and abdominal sensors which may measure breathing pattern and extension of the chest and form corresponding sensor information that may be transmitted to a controller of the system for further processing. Similarly, one or more electrocardiography (ECG) sensors may sense physiological parameters of a chest of the patient and form corresponding ECG information that may be provided to the controller for further processing. One or more flow sensors may monitor inspiratory and expiratory flow to, or from, the patient, respectively, and form corresponding flow information that may be provided to the controller of the system. The process may also obtain carbon dioxide (CO2) information from a CO2 sensor of the system. Further, PPG sensors may measure variability in stroke volume and/or cardiac output using optical measurement methods where a light source and a photodetector are used at the surface of skin, for example, to measure the volumetric variations of blood circulation. The various sensor information may be collected by the controller continuously in real time or at periodic or non-periodic intervals throughout the process.

After completing act 305, the process may continue to act 307 during which the process may analyze sensor information from one or more of the chest and abdominal sensors and determine breathing pattern and an extension of the chest (e.g., of the patient) based upon this sensor information.

After completing act 307, the process may continue to act 309 during which the process may analyze the ECG sensor information to determine one or more of heart rate (HR) and heart rate variability (HRV), the latter of which is a variation in the time interval between heartbeats (which may also be known as cycle-length variability, R-R variability, and/or heart period variability).

After completing act 309, the process may continue to act 311 during which the process may analyze the (air) flow information obtained from the flow sensors to determine inspiratory and expiratory flow patterns. During this act, the process may analyze the flow information and distinguish between passive and active expiration. It should be appreciated that increased hyperinflation leads to a forced outflow of air in the passive expiration cycle. The process may also analyze the information obtained from the chest sensor to determine whether the patient is in hyperinflation.

After completing act 311, the process may continue to act 315 during which the process may receive the CO2 sensor information and may determine CO2 concentration in the expiratory air flow to estimate and/or otherwise determine the gas trapping the in lung (alveoli). The CO2 sensor may be part of a capnograph of the system which may analyze the expiratory airflow to determine the CO2 in the expiratory airflow. The CO2 in the expiratory airflow is linked to gas trapping in the alveoli and insufficient CO2 clearance.

After completing act 315, the process may continue to act 317 during which the process may analyze the (air) flow pattern to determine the respiration effort in active expiration. As hyperinflation increases, this may lead to a forced outflow of air in the expiration cycle. COPD patients typically compensate for the collapse of bronchi/alveoli by intrinsic positive end-expiratory pressure (PEEP). This compensation may be sufficient when the patient is awake but can often fail during sleep. By analyzing the flow, an expiratory flow limitation (EFL) may be detected which is specific for the collapse of bronchi/alveoli. To measure a threshold of EFL (when the flow limitation starts) pressure maneuvers may be employed, then the system may determine an intrinsic PEEP. However, even without determining an exact value for PEEP, the process may determine that a flow limitation exists.

After completing act 317, the process may continue to act 319 during which the process may analyze cardiac data included in the ECG sensor information or other suitable cardiac sensor(s) such as PPG and determine a change in cardiac performance, such as cardiac output, ejection fraction and/or stroke volume. This change is then correlated to the changes in the HRV and stroke volume (SV) as a surrogate for increased sympathetic activity. These changes to the HRV and SV may be referred to as (ΔHRV) and (ΔSV), respectively. The stroke volume (SV) is a volume of blood pumped from the left ventricle per beat. The change in cardiac performance may be detected within one or a few heart cycles, hyperinflation changes the venous return flow and the cardiac preload/afterload. Sensors (in heart and central vascular structures) may sense it immediately where the sympathetic outflow is a direct consequence.

After completing act 319, the process may continue to act 321 during which the process may, in the time domain, determine the rise time of expiration (RTE). Accordingly, the process may analyze, in the time domain, the flow information to determine the rise time of the expiration as described in connection with act 215.

After completing act 321, the process may continue to act 323 during which the process may determine whether hyperinflation is critical. Accordingly, if it is determined that hyperinflation is critical, the process may continue to act 325. However, if it is determined that hyperinflation is not critical, the process may repeat act 305. The process may determine that hyperinflation is critical if the RTE is less than the threshold rise time (Trte) as discussed above with respect to act 217.

During act 325, the process may determine a current therapy mode. Accordingly, if the current (therapy) mode is CPAP or APAP, the process may continue to act 327. However, if it is determined that the current mode is BiPAP, the process may continue to acts 333 and 339 which may be performed serially or in parallel with each other. Although, APAP, CPAP, and BiPAP modes are illustrated for the sake of clarity in the current embodiments, other therapy modes are also envisioned and may have processes flows defined for them. In accordance with embodiments of the present system, all modes (e.g., APAP, BiPAP, CPAP, etc.) may provide benefits to all users through enhanced pressure control features, where BiPAP may include additional pressure control features than may be offered in some other modes such as CPAP and/or APAP.

During act 327, the process may change the C-flex, A-flex, and/or Bi-flex (B-flex) settings to corresponding Cflex, Aflex, and Bflex thresholds values, respectively, to ease the outflow of air during expiration. For example, similar to that described in connection with act 221, the process may, depending upon system settings, decrement the Aflex settings, by, for example, a desired Aflex decrement value (Adec) of one (although other values are also envisioned as may be set forth in the SSI). Thus, a current Aflex setting may be decremented by Adec such that (Aflex (next) = Aflex (current) - Adec). Similarly, the process may, depending upon system settings, decrement the Bflex settings, by, for example, a desired Bflex decrement value (Bdec) of one (although other values are also envisioned as may be set forth in the SSI). Thus, a current Bflex setting may be decremented by Bdec such that (Bflex (next) = Bflex (current) - Bdec). Similarly, the process may, depending upon system settings, decrement the Cflex settings, by, for example, a desired Cflex decrement value (Cdec) of one (although other values are also envisioned as may be set forth in the SSI). Thus, a current Cflex setting may be decremented by Cdec such that (Cflex (next) = Cflex (current) - Cdec). Accordingly, when changed C-flex, A-flex, and B-flex, pressures may be reduced by one in the present example.

It is also envisioned that the settings of C-flex, A-flex, and/or B-flex may be set to corresponding specified predetermined threshold values. For example, the C-flex setting be set to a Cflex threshold value (Ct) which may correspond to a desired pressure drop that may reduce the air pressure supplied by the system during each exhale (e.g., expiration) by the patient. The Cflex threshold value (Ct) may be an integer, such as two, in the present embodiments although over values are also envisioned and may be set forth in the SSI. With regard to the A-flex setting, this may be set to the Aflex threshold value (At) which may correspond to a pressure drop that may reduce the air pressure supplied by the system during each inhale (e.g., inhalation) by the patient. The Aflex threshold value may be an integer, such as two, in the present embodiments although over values are also envisioned and may be set forth in the SSI. With regard to the B-flex setting, this may be set to the Bflex threshold value (Bt) which may correspond to a pressure drop that may reduce the air pressure supplied by the system during each inhale (e.g., inhalation) by the patient. The Bflex threshold value may be an integer, such as two, in the present embodiments, although over values are also envisioned and may be set forth in the SSI.

After completing act 327, the process may continue to act 329 during which the process may determine whether hyperinflation is critical. Accordingly, if it is determined that hyperinflation is critical, the process may continue to act 331. However, if it is determined that hyperinflation is not critical, the process may continue to act 350. The process may determine that hyperinflation is critical if the RTE is less than the threshold rise time (Trte) as discussed above with respect to act 217.

During act 331 and similar to act 225, the process may shorten the time period for the pressure support during inspiration to decrease the tidal volume for M consecutive breath cycles, where M is an integer and may be set to 10 in the current embodiments. However, other values for M are also envisioned and may be set forth in the SSI. After completing act 331, the process may repeat act 329. Accordingly, hyperinflation may be monitored, and the above protocol may be continued until the target for a decreased chest inflation is reached and the controller 120 determines that hyperinflation is not or (no longer) critical.

During act 350 and similar to act 227, the process may return to target values as were initially set before the current process detected that hyperinflation is critical. The target values may be stored in a memory of the system during the initialization (e.g., act 303) and/or may be obtained from the SSI. Accordingly, the process may access the memory and/or the SSI for these default values. The process may also store current values for one or more settings of the system such as pressure and duration (e.g., inspiratory pressure and duration as well as expiratory pressure and duration, values for A-flex, B-flex, C-flex, etc.) e.g., in the SMI within a memory of the system such that if critical hyperinflation is detected again, the process may (look up the SMI) and return to the values that eliminated or reduced the critical hyperinflation (e.g., as first detected during act 323). In this regard, embodiments of the present system may provide sensing systems for hyperinflation, arousal, and airway pressure/resistance, and a controller with a learning and feedback loop for optimal (personalized) pressure adaptation, to improve the apnea hypopnea index (AHI) and sleep quality of the patient. Then, upon or in response to detecting critical hyperinflation, the system may immediately switch to the previous values that were found to eliminate or reduce the critical hyperinflation. Thus, the process may learn these values and store them in a memory of the system such as in the SMI for later use. After completing act 350, the process may continue to act 351 where it may end.

During act 333, the process may reduce expiratory pressure by a threshold pressure value (J) (where J is an integer) for the N (where N is an integer and is equal to 5 in the current embodiments although other numbers are also envisioned) next expiratory cycles (or for a threshold time TT, depending upon system settings) to ease the outflow of air from the lungs of the patient and reduce the hyperinflation. Accordingly, a controller of the system may be operative to control the PAM (e.g., PAM 144 of the PAP 102 shown in FIG. 1) to reduce expiratory positive airway pressure (EPAP) by an amount as set forth by a threshold pressure value J. J may be set to a value or values which may reflect an amount the EPAP may be reduced such as by 0.10, 0.20, ... 0.50, etc. which may reflect a pressure reduction such as 10%, 20%, ... 50%, etc., respectively in the EPAP. However, in yet other embodiments, J may be set to other values and/or ranges of values. In yet other embodiments, the EPAP may be reduced in steps of 0.5 cmH₂0 which may be held for a threshold number of breath cycles (e.g., 5, in the present embodiments although other values are also envisioned), before the process may step down again in accordance with embodiments of the present system.

After completing act 333, the process may continue to act 335 during which the process may determine whether hyperinflation is critical. Accordingly, if it is determined that hyperinflation is critical, the process may continue to act 337. However, if it is determined that hyperinflation is not critical, the process may continue to act 350. The process may determine that hyperinflation is critical if the RTE is less than the threshold rise time (Trte) as discussed above with respect to act 217.

During act 337, the process may decrease the time period for the pressure support during inspiration to decrease the tidal volume (of inspiratory air) for a threshold number (e.g., M) of consecutive breath cycles. After completing act 337, the process may repeat act 335. Thus, hyperinflation will be monitored, and the above protocol will be continued until the target for a decreased chest inflation and being confirmed/validated by a change of rise time of expiration is reached.

During act 339 and similar to act 231, the process may reduce inspiration pressure by a threshold pressure value (J) (where J is an integer) for the N (where N is an integer and is equal to 5 in the current embodiments although other numbers are also envisioned) next inspiration cycles to decrease tidal volume and to reduce the hyperinflation. It is known that the effect of positive pressure on hyperinflation can be the opposite where increased positive pressure reduces hyperinflation. For example, at greater than or equal to 10 mm H₂O, the lung may be considered to be deflated compared to no PAP. Accordingly, a controller of the system may be operative to control the PAM 144 (e.g., control the PAM pressure and/or flow, and/or control regulators and/or valves, etc.) to reduce inspiratory positive airway pressure (IPAP) by an amount as set forth by a threshold pressure value J.

After completing act 339, the process may continue to act 341 during which the process may determine whether hyperinflation is critical. Accordingly, if it is determined that hyperinflation is critical, the process may continue to act 343. However, if it is determined that hyperinflation is not critical, the process may continue to act 350. The process may determine that hyperinflation is critical if the RTE is less than the threshold rise time (Trte) as discussed above with respect to act 217. Thus, If the decline of the inspiratory BiPAP pressure performed during act 339 is insufficient, the pressure protocol as described for the APAP therapy mode may be employed.

During act 343, the process may change the C-flex and/or A-flex settings to corresponding Cflex and Aflex thresholds values, respectively, to ease the outflow of air during expiration. For example, the process may, depending upon system settings, decrement the Aflex settings, by, for example, a desired Aflex decrement value (Adec) (as may be set forth in the SSI) of one, although other values are also envisioned. This act may be similar to act 221. Accordingly, reference is made to act 221 for a further description.

After completing act 343, the process may continue to act 347 during which the process may determine whether hyperinflation is critical. Accordingly, if it is determined that hyperinflation is critical, the process may continue to act 349. However, if it is determined that hyperinflation is not critical, the process may continue to act 350. The process may determine that hyperinflation is critical if the RTE is less than the threshold rise time (Trte) as discussed above with respect to act 217.

During act 349, the process may reduce period of pressure support in the inspiration cycle by a timely step value (TJ) (where TJ is an integer and may be equal to 100 ms in the present embodiments, although other values are also envisioned as may be set in the SSI) for the N (where N is an integer and is equal to 5 in the current embodiments although other numbers are also envisioned) next inspiration cycles to decrease tidal volume and to reduce the hyperinflation. Accordingly, a controller of the system may be operative to control the PAM (e.g., the PAM 144 of the PAP device 100 shown in FIG. 1) to reduce the period of pressure support in the inspiration cycle by the timely step value (TJ). After completing act 349, the process may repeat act 347. Accordingly, hyperinflation may be monitored, and the above protocol may be continued until the target for a decreased chest inflation is reached.

In COPD patients, a certain hyperinflation and the PEEP is essential to counteract the collapse in expiration. Accordingly, during method 300 the system may monitor for a progressing collapse in the bronchi of the patient (by analyzing the air flow pattern as discussed during act 317 for EFL) and if a progressing collapse of the bronchi is detected (as determined through analysis of the EFL, etc.), expiratory pressure may be increased by a threshold amount (as may be set forth in the SSI) to provide for proper expiration.

Accordingly, chest and/or abdominal sensors may monitor the decrease in hyperinflation, and when chest inflation may once again reach the threshold, the BIPAP device settings may be set back to predefined values. Thus, a learning loop may be employed.

With reference to FIG. 4, in operation, the process 400 may start during act 401 and then proceed to act 403 where it may initialize the PAP device 102, etc., by performing an initialization routine. During initialization, the process may load SSI and may set system settings in accordance with the SSI. The system may set pressure settings (e.g., inhalation pressure, exhalation pressure, etc.) to default values. This act may be similar to act 203. Accordingly, reference is made to act 203 for further description of this act.

After completing act 403, the process may continue to act 405 during which the process may obtain sensor information from one or more sensors of the system, such as from chest and abdominal sensors, etc. which may measure breathing pattern and extension of the chest and form corresponding sensor information, that may be transmitted to a controller of the system for further processing. Similarly, one or more electrocardiography (ECG) sensors may sense physiological parameters of a chest of the patient and form corresponding ECG information that may be provided to the controller for further processing. One or more flow sensors may monitor inspiratory and expiratory flow to or from the patient, respectively, and form corresponding flow information that may be provided to the controller of the system. The process may also obtain CO2 information from a CO2 of the system. PPG sensors may measure variability in stroke volume and/or cardiac output. The sensor information may be collected by the controller continuously in real time or at periodic or non-periodic intervals.

After completing act 405, the process may continue to act 407 during which the process may analyze sensor information from one or more of the chest and abdominal sensors and determine a breathing pattern and an extension of the chest based upon this sensor information.

After completing act 407, the process may continue to act 409 during which the process may analyze the ECG sensor information to determine one or more of heart rate (HR) and heart rate variability (HRV) the latter of which is a variation in the time interval between heartbeats (aka, cycle-length variability, R-R variability, and heart period variability).

After completing act 409, the process may continue to act 411 during which the process may analyze the PPG information from the PPG sensors and determine variability in stroke volume and ejection fraction. For example, cardiac output may be determined via pulse wave analysis of the PPG signals contained in the PPG information.

After completing act 411, the process may continue to act 415 during which the process may analyze the (air) flow information obtained from the flow sensors to determine inspiratory and expiratory flow patterns. During this act, the process may analyze the flow information and distinguish between passive and active expiration. It should be appreciated that increased hyperinflation leads to a forced outflow of air in the passive expiration cycle. The process may perform pulse wave analysis of a PPG sensor signal to aid in the determination of one or more of cycle-length variability, R-R variability, and heart period variability, the heart rate (HR) and heart rate variability (HRV).

After completing act 415, the process may continue to act 417 during which the process may analyze ECG and PPG sensor information to determine the implication of PAP pressure therapy on venous return flow (VRF), stroke volume (SV) and/or cardiac output (CO) (which may be derived from one or more of the determined HR, HRV, and PPG sensor information). A change in venous return flow will change HR/HRV caused by the activation of baroreceptor in the cardiac right atrium. For example, the process may perform pulse wave analysis of information obtained from one or more of the PPG sensors and/or ECG to determine one or more of CO, SV, and EF. With regard to the implication of PAP therapy on VRF, SV, and CO, if one or more of these decreases by more than a threshold value such as 10% (although other threshold values are also envisioned), an intervention (e.g., to decrease critical hyperinflation) may be determined to be desired and performed by the system in accordance with system settings.

It would be appreciated that VRF may represent the inflow (into the heart); and SV may represent the outflow (from the heart). Averaged over time VRF may generally equal SV. However, during the transition from inspiration to expiration and vice versa, it can differ due to the modulation of the pressure in the lung. This is also the respiration modulated heart rate variability: and in the present embodiments may represent the hyperinflation related HRV. HRV in hyperinflation may be stronger than in shallow breathing, this information may be indicative of hyperinflation. Hyperinflation may change VRF, the physiological response is an HR/HRV change triggered by baroreceptors. Consequently, the SV will change (caused by a change of VRF). The change of SV is visible in a PPG signal (e.g., included in PPG sensor information generated by a PPG sensor of the system). Accordingly, the process may analyze the PPG signal to determine a change in SV.

After completing act 417, the process may continue to act 419 during which the process may determine if venous return flow (VRF) is less than a threshold for venous return flow (Tvrf). Accordingly, when it is determined that the venous return flow (VRF) is less than the threshold for venous return flow (Tvrf), the process may continue to act 421. However, when it is determined that the venous return flow (VRF) is not less than (e.g., is equal to or greater than) the threshold for venous return flow (Tvrf), the process may repeat act 405. The threshold for venous return flow (Tvrf) may be obtained from a memory of the system such as from the SSI.

During act 421, the process may determine if stroke volume (SVOL) is less than a threshold for stroke volume (Tsvol). Accordingly, when it is determined that the stroke volume (SV) is less than the threshold for stroke volume (Tsvol), the process may continue to act 423. However, when it is determined that the stroke volume (SVOL) is not less than (e.g., is equal to or greater than) the threshold for stroke volume (Tsvol), the process may repeat act 405. With regard to the value of Tsvol, this value may be set by the system based upon a previous value of stroke volume (SVOLpv) less a threshold value such as 10% (e.g., 0.10) although other values are also envisioned. Accordingly, Tsvol may be defined as SVOLpv*(1-threshold value) which, in the present embodiments assuming that Tsvol is equal to 0.10, then Tsvol may be defined as SVOLpv*(1-0.10) which may be equal to Tsvol = (SVOLpv*0.90). The threshold for stroke volume (Tsvol) may be determined dynamically with the threshold value (e.g., 0.01 or 10% in the present embodiments, although other values are also envisioned) obtained from a memory of the system such as from the SSI.

During act 423, the process may decrease current PAP pressure settings by a default pressure value Pd for N breath cycles, where N is an integer and may be set to 5 for the present embodiments. Pd may be defined by the SSI and may be equal to 0.5cmH₂O although other values are also envisioned. In yet other embodiments the pressure may be dropped by a predefined percentage value if desired as may be set in the SSI.

After completing act 423, the process may continue to act 425 during which the process may determine when venous return flow (VRF) is less than the threshold for venous return flow (Tvrf). Accordingly, when it is determined that the venous return flow (VRF) is less than the threshold for venous return flow (Tvrf), the process may continue to act 427. However, when it is determined that the venous return flow (VRF) is not less than (e.g., is equal to or greater than) the threshold for venous return flow (Tvrf), the process may continue to act 429.

During act 427, as in act 421, the process may determine if stroke volume (SVOL) is less than the threshold for stroke volume (Tsvol). Accordingly, when it is determined that the stroke volume (SV) is less than the threshold for stroke volume (Tsvol), the process may repeat act 423. However, when it is determined that the stroke volume (SVOL) is not less than (e.g., is equal to or greater than) the threshold for stroke volume (Tsvol), the process may continue to act 429.

During act 429, the process may return the pressure settings back to the default value as may be set forth in the SSI. At this point, one or more of the venous returns and the stroke volume are rebalanced and the pressure settings may be reset back to their respective default values.

With regard to repeating act 423, this act may be repeated when the venous return and the stroke volume are not rebalanced. In some embodiments, the pressures, once dropped, may be held steady while in other embodiments the pressure settings may be continually dropped until the venous return and the stroke volume are rebalanced. After completing act 429, the process may continue to act 431 where it may end.

With reference to FIG. 5, in operation, the process 500 may start during act 501 and continue to act 503 where it may perform flow analysis. Accordingly, the process may analyze sensor information obtained from one or more sensors of the system such as from a flow sensor of a PAP device or a flow sensor in the mask (e.g., of the patient interface that may be coupled to the patient's face) to determine RTE. The process may further determine whether RTE decreases and, if so, validate whether this is cause by change breathing pattern that is specific to hyperinflation. After completing act 503, the process may continue to act 505.

During act 505, the process may perform a sympathetic response study which may include an electrophysiological analysis. The process may sense whether a sympathetic response of hyperinflation (e.g., using ECG as a primary sensor) is present. As hyperinflation impedes the venous return flow, the sympathetic response of an alteration of VRF (and a hemodynamic parameter in general) is a change of HR and HRV triggered by baroreceptors in the heart. The process may further employ alternative secondary sensors to provide additional sensory information, such as from one or more of EEG, EMG, and/or bioimpedance sensors, to sense the sympathetic response. Then, the process may validate whether the sympathetic response is critical to trigger an arousal. After completing act 505, the process may continue to act 507.

During act 507, the process may determine physiological implications including hemodynamic alterations, and validation of whether the hemodynamic change is critical and specific for a ventilation/circulation mismatch (e.g., decreased blood flow, increase CO2) triggering chemoreceptors and baroreceptors. The process may validate and cross check whether the HR, HRV change is caused by a VRF change (to distinguish from other causes). Accordingly, the process may acquire a PPG signal (e.g., from a PPG sensor which may be considered a primary sensor in the present embodiments) to validate the blood flow changes, where a change/decrease in VRF will change/decrease SV in the next heart cycle(s). During this act, the process may further acquire sensor information from secondary sensors such as from one or more of the chest and abdominal sensors (e.g., chest band sensors aka chest belt(s)) and a cardiobalistography (CBG) sensor. After completing act 507, the process may continue to act 509.

During act 509, the process may determine a conclusion. Accordingly, the process may validate whether HR and PPG changes are specific for hyperinflation, and may then adapt a therapy to reduce or eliminate hyperinflation. The process may further render information related to the conclusion on a rendering device of the system for the convenience of the clinician. After competing act 509, the process may continue to act 511.

During act 511, the process may provide decision support to adapt PAP device settings. The process may quantify the HR and/or HRV changes, and if changes are determined to be critical then process may determine to adapt PAP device settings. The process may further determine an amplitude of the PPG signal and may quantify the amplitude of the PPG signal and relative changes in the PPG amplitude. For example, the process may determine whether the PPG is greater than a threshold, and when in the affirmative then the process may adapt PAP device settings. After completing act 511, the process may continue to act 515 where it may end.

Embodiments of the present system may be integrated into various PAP solutions such as APAP, BiPAP, and CPAP, and may be operated in home settings as well as in institutional settings such as in, hospitals, nursing homes, rehabilitation centers, and/or the like.

As operations may be automated, hyperinflation may be controlled resulting in decreased sympathetic nervous system activation which may enhance patient sleep, comfort, and use, which may lead to better outcomes for the patient.

Embodiments of the present system may be compatible with obstructive sleep apnea (OSA) devices, therapy selection and adaptation (e.g., CPAP, pharma, etc.). It is also envisioned that embodiments of the present system may be compatible with non-invasive mechanical ventilation system and devices for COPD management therapy.

The processes 200, 300, 400, 500 may be performed using one or more processors, computers, controllers, etc., communicating over a network and may obtain information from, and/or store information to, one or more memories which may be local and/or remote from each other. Accordingly, as referenced herein, such as "the process may ... " refers generally to one or more controllers performing an operation in accordance with embodiments of the present system, though in instances may refer to the operating process (e.g., "the process may start ..."). The processes 200, 300, 400, 500 may include one of more of the following acts. In accordance with embodiments of the present system, the acts of the processes 200, 300, 400, 500 may be performed using a controller operating in accordance with embodiments of the present system. Further, one or more of these acts may be combined and/or separated into sub-acts, or performed serially or in parallel, as may be desired. Further, one or more of these acts may be skipped depending upon settings. For the sake of clarity, the process may be described with reference to a single PAP and a single patient case. However, without limitation, it should be understood that the process may employ a plurality of PAPs and a plurality of patient cases each of which may include a separate workflow such as a sub-workflow.

Further variations of the present system would readily occur to a person of ordinary skill in the art and are encompassed by the following claims.

Finally, the above discussion is intended to be merely illustrative of the present system and should not be construed as limiting the appended claims to any particular embodiment or group of embodiments. Thus, while the present system has been described with reference to exemplary embodiments, it should also be appreciated that numerous modifications and alternative embodiments may be devised by those having ordinary skill in the art including using features that are described with regard to a given embodiment with other envisioned embodiments without departing from the broader and intended scope of the present system as set forth in the claims that follow. In addition, any section headings included herein are intended to facilitate a review but are not intended to limit the scope of the present system, as claimed in the appended claims. In addition, the specification and drawings are to be regarded in an illustrative manner and are not intended to limit the scope of the appended claims.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function;
e) any of the disclosed elements may be comprised of hardware portions (e.g., including discrete and integrated electronic circuitry), software portions (e.g., computer programming), and any combination thereof;
f) hardware portions may be comprised of one or both of analog and digital portions;
g) any of the disclosed devices, features and/or portions thereof may be combined together or separated into further portions unless specifically stated otherwise;
h) no specific sequence of acts or steps is intended to be required unless specifically indicated; and
i) the term "plurality of" an element includes two or more of the claimed element, and does not imply any particular range of number of elements; that is, a plurality of elements may be as few as two elements, and may include an immeasurable number of elements.

## Claims

1. A positive airway pressure (PAP) (100) therapy device, comprising:
at least one controller (120, 190, 194);
a flow generator (144) configured to provide an airflow to a user under control of the at least one controller (120, 190, 194);
a plurality of sensors (114) including at least one electrocardiogram (ECG) sensor, at least one photoplethysmogram (PPG) sensor, and at least one flow sensor,
wherein the at least one flow sensor is configured to determine inspiratory and expiratory flow patterns and form corresponding inspiratory and expiratory flow information, and
wherein the at least one ECG sensor is configured to sense physiological parameters of a user and form corresponding ECG sensor information,
wherein the at least one PPG sensor is configured to measure variability in stroke volume (SV), ejection fraction (EF), and/or cardiac output (CO);
the at least one controller (120, 190, 194) configured to:
control the flow generator (144) to provide therapeutic air assist to the user in accordance with an initial setting mode;
distinguish active and passive expiration based on the inspiratory and expiratory flow information;
determine a rise time of expiration (RTE) based upon the expiratory flow information, wherein the rise time of expiration (RTE) is indicative of how fast the user exhales, wherein the shorter the expiration rise time (RTE) the stronger the hyperinflation;
compare the rise time of expiration (RTE) to a threshold rise time of expiration value (Trte) to determine whether the rise time of expiration (RTE) is less than the threshold rise time of expiration value (Trte);
determine hyperinflation when the at least one controller (120, 190, 194) determines that the rise time of expiration (RTE) is less than the threshold rise time of expiration value (Trte);
following determining the hyperinflation, determine arousals based on the ECG sensor information;
determine a decreased cardiac output or stroke volume and/or ejection fraction following determining the hyperinflation;
determine the hyperinflation is critical based on the rise time of expiration (RTE) being less than the threshold rise time of expiration value (Trte), and the arousals, and the decreased cardiac output or stroke volume and/or ejection fraction;
control the flow generator (144) to operate in accordance with a hyperinflation mode to reduce therapeutic air assist to the user when the at least one controller determines that the hyperinflation is critical; and
control the flow generator (144) to decrease expiration pressure by a threshold pressure value when the PAP therapy device is in the hyperinflation mode.

2. The PAP therapy device according to claim 1, wherein the at least one controller (120, 190, 194) is further configured to control the flow generator (144) to decrease duration of pressure support during inspiration cycle for N breath cycles by a threshold time value when in the hyperinflation mode, where N is an integer.

3. The PAP therapy device according to any of the preceding claims, wherein the at least one controller (120, 190, 194) is further configured to control the flow generator (144) to reduce at least one of duration of pressure support during inspiration cycle and inspiration pressure by corresponding threshold amounts when in the hyperinflation mode.

4. The PAP therapy device according to any of the preceding claims, wherein the plurality of sensors (114) includes a chest sensor and an abdominal area sensor configured to measure a breathing pattern and extension of a chest of the user and form corresponding chest inflation sensor information (CISI).

5. The PAP therapy device according to claim 4, wherein the at least one controller (120, 190, 194) is further configured to compare the chest inflation sensor information (CISI) to a threshold chest inflation value (Tci).

6. The PAP therapy device according to claim 5, wherein when in the hyperinflation mode, the at least one controller is further configured to determine the hyperinflation is not critical when the CISI is determined to be less than the Tci.

7. The PAP therapy device according to claim 6, wherein the at least one controller (120, 190, 194) is further configured to control the flow generator (144) to return to operation in accordance with the initial setting mode to restore therapeutic air assist to the user in accordance with the initial setting mode, when the at least one controller (120, 190, 194) determines that the hyperinflation is not critical.

8. The PAP therapy device according to claim 7, wherein the at least one controller (120, 190, 194) is further configured to save in a memory (126) current hyperinflation mode operating parameters of the flow generator (144) prior to returning to operation in accordance with the initial setting mode from the hyperinflation mode.

9. The PAP therapy device according to claim 8, wherein when the at least one controller (120, 190, 194) determines that the hyperinflation is critical, the at least one controller is further configured to change operating parameters of the flow generator (144) to the saved current hyperinflation mode operating parameters.

10. The PAP therapy device according to any of the preceding claims, wherein the at least one controller (120, 190, 194) is further configured to determine the hyperinflation is not critical when the at least one controller (120, 190, 194) determines that the rise time of expiration (RTE) is not less than the threshold rise time of expiration (Trte).

11. The PAP therapy device according to claim 6, wherein the at least one controller (120, 190, 194) is further configured to control the flow generator (144) to return to operation in accordance with the initial setting mode to restore therapeutic air assist to the user in accordance with the initial setting mode, when the at least one controller (120, 190, 194) determines that hyperinflation is not critical.

## Patentansprüche

1. Vorrichtung zur Therapie mit positivem Atemwegsdruck (PAP) (100), umfassend:
mindestens eine Steuereinheit (120, 190, 194);
einen Strömungsgenerator (144), der dazu konfiguriert ist, einem Benutzer unter der Steuerung der mindestens einen Steuereinheit (120, 190, 194) einen Luftstrom bereitzustellen;
eine Vielzahl von Sensoren (114), einschließlich mindestens eines Elektrokardiogramm (EKG)-Sensors, mindestens eines Photoplethysmogramm (PPG)-Sensors und mindestens eines Strömungssensors,
wobei der mindestens eine Strömungssensor dazu konfiguriert ist, inspiratorische und exspiratorische Strömungsmuster zu bestimmen und entsprechende inspiratorische und exspiratorische Strömungsinformationen zu bilden, und
wobei der mindestens eine EKG-Sensor dazu konfiguriert ist, physiologische Parameter eines Benutzers zu erfassen und entsprechende EKG-Sensorinformationen zu bilden,
wobei der mindestens eine PPG-Sensor dazu konfiguriert ist, die Variabilität des Schlagvolumens (SV), der Ejektionsfraktion (EF) und/oder des Herzzeitvolumens (CO) zu messen;
wobei die mindestens eine Steuereinheit (120, 190, 194) dazu konfiguriert ist:
den Strömungsgenerator (144) zu steuern, um dem Benutzer gemäß einem anfänglichen Einstellungsmodus therapeutische Luftunterstützung bereitzustellen;
zwischen aktiver und passiver Exspiration basierend auf den inspiratorischen und exspiratorischen Strömungsinformationen zu unterscheiden;
eine exspiratorischen Anstiegszeit (RTE) basierend auf den exspiratorischen Strömungsinformationen zu bestimmen, wobei die exspiratorische Anstiegszeit (RTE) anzeigt, wie schnell der Benutzer ausatmet, wobei die Überblähung umso stärker ist, je kürzer die exspiratorische Anstiegszeit (RTE) ist;
die exspiratorische Anstiegszeit (RTE) mit einem Schwellenwert der exspiratorischen Anstiegszeit (Trte) zu vergleichen, um zu bestimmen, ob die exspiratorische Anstiegszeit (RTE) kleiner ist als der Schwellenwert der exspiratorischen Anstiegszeit (Trte).
eine Überblähung zu bestimmen, wenn die mindestens eine Steuereinheit (120, 190, 194) bestimmt, dass die exspiratorische Anstiegszeit (RTE) kleiner ist als der Schwellenwert der exspiratorischen Anstiegszeit (Trte);
nach Bestimmen der Überblähung, die Arousal-Werte basierend auf den EKG-Sensorinformationen zu bestimmen;
nach Bestimmen der Überblähung, ein verringertes Herzzeitvolumens oder Schlagvolumen und/oder eine verringerte Ejektionsfraktion zu bestimmen;
zu bestimmen, dass die Überblähung kritisch ist, basierend auf der exspiratorischen Anstiegszeit (RTE), die kürzer ist als der Schwellenwert der exspiratorischen Anstiegszeit (Trte), und den Arousal-Werten und dem verringerten Herzzeitvolumen oder Schlagvolumen und/oder der verringerten Ejektionsfraktion;
den Strömungsgenerators (144) zu steuern, sodass dieser gemäß einem Überblähungsmodus arbeitet, um die therapeutische Luftunterstützung für den Benutzer zu reduzieren, wenn die mindestens eine Steuereinheit bestimmt, dass die Überblähung kritisch ist; und
den Strömungsgenerator (144) zu steuern, um den Exspirationsdruck um einen Schwellendruckwert zu verringern, wenn sich die PAP-Therapievorrichtung im Überblähungsmodus befindet.

2. PAP-Therapievorrichtung nach Anspruch 1, wobei die mindestens eine Steuereinheit (120, 190, 194) weiter dazu konfiguriert ist, den Strömungsgenerator (144) so zu steuern, dass im Überblähungsmodus die Dauer der Druckunterstützung während des Inspirationszyklus für N Atemzyklen um einen Schwellenzeitwert verringert wird, wobei N eine ganze Zahl ist.

3. PAP-Therapievorrichtung nach einem der vorstehenden Ansprüche, wobei die mindestens eine Steuereinheit (120, 190, 194) weiter dazu konfiguriert ist, den Strömungsgenerator (144) so zu steuern, dass im Überblähungsmodus mindestens eines von der Dauer von Druckunterstützung während des Inspirationszyklus und Inspirationsdruck um entsprechende Schwellenbeträge reduziert wird.

4. PAP-Therapievorrichtung nach einem der vorstehenden Ansprüche, wobei die Vielzahl von Sensoren (114) einen Brustkorbsensor und einen Bauchraumsensor beinhaltet, die dazu konfiguriert sind, ein Atemmuster und eine Ausdehnung eines Brustkorbs des Benutzers zu messen und entsprechende Brustkorbaufblähungs-Sensorinformationen (CISI) zu bilden.

5. PAP-Therapievorrichtung nach Anspruch 4, wobei die mindestens eine Steuereinheit (120, 190, 194) weiter dazu konfiguriert ist, die Brustkorbaufblähungs-Sensorinformationen (CISI) mit einem Brustkorbaufblähungs-Schwellenwert (Tci) zu vergleichen.

6. PAP-Therapievorrichtung nach Anspruch 5, wobei im Überblähungsmodus die mindestens eine Steuereinheit weiter dazu konfiguriert ist, zu bestimmen, dass die Überblähung nicht kritisch ist, wenn bestimmt wird, dass die CISI kleiner als die Tci sind.

7. PAP-Therapievorrichtung nach Anspruch 6, wobei die mindestens eine Steuereinheit (120, 190, 194) weiter dazu konfiguriert ist, den Strömungsgenerator (144) so zu steuern, dass er den Betrieb gemäß dem anfänglichen Einstellungsmodus wieder aufnimmt, um die therapeutische Luftunterstützung für den Benutzer gemäß dem anfänglichen Einstellungsmodus wiederherzustellen, wenn die mindestens eine Steuereinheit (120, 190, 194) bestimmt, dass die Überblähung nicht kritisch ist.

8. PAP-Therapievorrichtung nach Anspruch 7, wobei die mindestens eine Steuereinheit (120, 190, 194) weiter dazu konfiguriert ist, aktuelle Betriebsparameter des Strömungsgenerators (144) im Überblähungsmodus in einem Speicher (126) zu speichern, bevor aus dem Überblähungsmodus wieder der Betrieb gemäß dem anfänglichen Einstellungsmodus aufgenommen wird.

9. PAP-Therapievorrichtung nach Anspruch 8, wobei, wenn die mindestens eine Steuereinheit (120, 190, 194) bestimmt, dass die Überblähung kritisch ist, die mindestens eine Steuereinheit weiter dazu konfiguriert ist, die Betriebsparameter des Strömungsgenerators (144) auf die gespeicherten aktuellen Betriebsparameter im Überblähungsmodus zu ändern.

10. PAP-Therapievorrichtung nach einem der vorstehenden Ansprüche, wobei die mindestens eine Steuereinheit (120, 190, 194) weiter dazu konfiguriert ist, zu bestimmen, dass die Überblähung nicht kritisch ist, wenn die mindestens eine Steuereinheit (120, 190, 194) bestimmt, dass die exspiratorische Anstiegszeit (RTE) nicht kleiner als der Schwellenwert der exspiratorischen Anstiegszeit (Trte) ist.

11. PAP-Therapievorrichtung nach Anspruch 6, wobei die mindestens eine Steuereinheit (120, 190, 194) weiter dazu konfiguriert ist, den Strömungsgenerator (144) so zu steuern, dass er den Betrieb gemäß dem anfänglichen Einstellungsmodus wieder aufnimmt, um die therapeutische Luftunterstützung für den Benutzer gemäß dem anfänglichen Einstellungsmodus wiederherzustellen, wenn die mindestens eine Steuereinheit (120, 190, 194) bestimmt, dass Überblähung nicht kritisch ist.

## Revendications

1. Dispositif de thérapie par ventilation en pression positive (PAP) (100), comprenant :
au moins un dispositif de commande (120, 190, 194) ;
un générateur de flux (144) configuré pour fournir un flux d'air à un utilisateur sous la commande du au moins un dispositif de commande (120, 190, 194) ;
une pluralité de capteurs (114) incluant au moins un capteur d'électrocardiogramme (ECG), au moins un capteur de photopléthysmogramme (PPG) et au moins un capteur de débit,
dans lequel le au moins un capteur de débit est configuré pour déterminer des motifs de débit inspiratoire et expiratoire et former des informations de débit inspiratoire et expiratoire correspondantes, et
dans lequel le au moins un capteur ECG est configuré pour détecter les paramètres physiologiques d'un utilisateur et former des informations de capteur ECG correspondantes,
dans lequel le au moins un capteur PPG est configuré pour mesurer la variabilité du volume systolique (SV), de la fraction d'éjection (EF), et/ou du débit cardiaque (CO) ;
le au moins un dispositif de commande (120, 190, 194) étant configuré pour :
commander le générateur de flux (144) pour fournir une assistance d'air thérapeutique à l'utilisateur selon un mode de réglage initial ;
distinguer l'expiration active et l'expiration passive sur la base des informations de débit inspiratoire et expiratoire ;
déterminer un temps de montée d'expiration (RTE) sur la base des informations de débit expiratoire, dans lequel le temps de montée d'expiration (RTE) indique la vitesse à laquelle l'utilisateur expire, dans lequel plus le temps de montée d'expiration (RTE) est court, plus l'hyperinflation est forte ;
comparer le temps de montée d'expiration (RTE) à une valeur seuil de temps de montée d'expiration (Trte) pour déterminer si le temps de montée d'expiration (RTE) est inférieur à la valeur seuil de temps de montée d'expiration (Trte) ;
déterminer l'hyperinflation lorsque le au moins un dispositif de commande (120, 190, 194) détermine que le temps de montée d'expiration (RTE) est inférieur à la valeur seuil de temps de montée d'expiration (Trte) ;
après avoir déterminé l'hyperinflation, déterminer les réveils sur la base des informations de capteur ECG ;
déterminer une diminution du débit cardiaque ou du volume systolique et/ou de la fraction d'éjection après détermination de l'hyperinflation ;
déterminer si l'hyperinflation est critique sur la base du temps de montée d'expiration (RTE) étant inférieur à la valeur seuil de temps de montée d'expiration (Trte), des réveils et de la diminution du débit cardiaque ou du volume systolique et/ou de la fraction d'éjection ;
commander le générateur de flux (144) pour qu'il fonctionne conformément à un mode d'hyperinflation afin de réduire l'assistance d'air thérapeutique à l'utilisateur lorsque le au moins un dispositif de commande détermine que l'hyperinflation est critique ; et
commander le générateur de flux (144) pour diminuer la pression d'expiration d'une valeur seuil de pression lorsque le dispositif de thérapie PAP est en mode hyperinflation.

2. Dispositif de thérapie PAP selon la revendication 1, dans lequel le au moins un dispositif de commande (120, 190, 194) est en outre configuré pour commander le générateur de flux (144) afin de diminuer la durée de l'assistance de pression pendant le cycle d'inspiration pour N cycles respiratoires d'une valeur seuil de temps en mode hyperinflation, où N est un nombre entier.

3. Dispositif de thérapie PAP selon l'une quelconque des revendications précédentes, dans lequel le au moins un dispositif de commande (120, 190, 194) est en outre configuré pour commander le générateur de flux (144) afin de réduire au moins l'une parmi la durée de l'assistance de pression pendant le cycle d'inspiration et la pression d'inspiration par des quantités seuils correspondantes en mode hyperinflation.

4. Dispositif de thérapie PAP selon l'une quelconque des revendications précédentes, dans lequel la pluralité de capteurs (114) inclut un capteur de poitrine et un capteur de zone abdominale configurés pour mesurer un motif de respiration et l'inflation thoracique de l'utilisateur et former des informations de capteur d'inflation thoracique (CISI) correspondantes.

5. Dispositif de thérapie PAP selon la revendication 4, dans lequel le au moins un dispositif de commande (120, 190, 194) est en outre configuré pour comparer les informations de capteur d'inflation thoracique (CISI) à une valeur seuil d'inflation thoracique (Tci).

6. Dispositif de thérapie PAP selon la revendication 5, dans lequel, en mode hyperinflation, le au moins un dispositif de commande est en outre configuré pour déterminer que l'hyperinflation n'est pas critique lorsqu'il est déterminé que les CISI sont inférieures à la Tci.

7. Dispositif de thérapie PAP selon la revendication 6, dans lequel le au moins un dispositif de commande (120, 190, 194) est en outre configuré pour commander le générateur de flux (144) afin qu'il revienne au fonctionnement selon le mode de réglage initial pour rétablir l'assistance d'air thérapeutique à l'utilisateur selon le mode de réglage initial, lorsque le au moins un dispositif de commande (120, 190, 194) détermine que l'hyperinflation n'est pas critique.

8. Dispositif de thérapie PAP selon la revendication 7, dans lequel le au moins un dispositif de commande (120, 190, 194) est en outre configuré pour sauvegarder dans une mémoire (126) les paramètres de fonctionnement actuels du mode d'hyperinflation du générateur de flux (144) avant de revenir au fonctionnement selon le mode de réglage initial à partir du mode d'hyperinflation.

9. Dispositif de thérapie PAP selon la revendication 8, dans lequel, lorsque le au moins un dispositif de commande (120, 190, 194) détermine que l'hyperinflation est critique, le au moins un dispositif de commande est en outre configuré pour modifier les paramètres de fonctionnement du générateur de flux (144) en fonction des paramètres de fonctionnement de mode d'hyperinflation actuels enregistrés.

10. Dispositif de thérapie PAP selon l'une quelconque des revendications précédentes, dans lequel le au moins un dispositif de commande (120, 190, 194) est en outre configuré pour déterminer que l'hyperinflation n'est pas critique lorsque le au moins un dispositif de commande (120, 190, 194) détermine que le temps de montée d'expiration (RTE) n'est pas inférieur au temps seuil de montée d'expiration (Trte).

11. Dispositif de thérapie PAP selon la revendication 6, dans lequel le au moins un dispositif de commande (120, 190, 194) est en outre configuré pour commander le générateur de flux (144) afin qu'il revienne au fonctionnement selon le mode de réglage initial pour rétablir l'assistance d'air thérapeutique à l'utilisateur selon le mode de réglage initial, lorsque le au moins un dispositif de commande (120, 190, 194) détermine que l'hyperinflation n'est pas critique.
